# EUROPEAN PATENT SPECIFICATION

(11) **EP 2 997 159 B1**
(45) Date of publication and mention of the grant of the patent: **24.07.2019**
(21) Application number: 14723796.0
(22) Date of filing: 13.05.2014
(51) Int. Cl.: C12Q 1/6886

(54) **METHOD FOR DETERMINING A HUMAN INDIVIDUAL'S PREDISPOSITION TO CONTRACT A HEMATOPOIETIC MALIGNANT MYELOID DISEASE**
VERFAHREN ZUR BESTIMMUNG DER PRÄDISPOSITION EINER PERSON FÜR EINE BÖSARTIGE MYELOISCHE HEMATOPOYETISCHE ERKRANKUNG
PROCÉDÉ DE DÉTERMINATION DE LA PRÉDISPOSITION D'UN INDIVIDU HUMAIN À CONTRACTER UNE MALADIE MYELOÏDE HEMATOPOÏETIQUE MALIGNE

(30) Priority: 13.05.2013 EP 13167411
(43) Date of publication of application: 23.03.2016
(73) Proprietor: Rheinisch-Westfälische Technische Hochschule (RWTH) Aachen, 52056 Aachen (DE)
(72) Inventor: BRUEMMENDORF, Tim Henrik, 52066 Aachen (DE); JOST, Edgar, B-4700 Eupen (BE); WAGNER, Wolfgang, 52078 Aachen (DE); WALENDA, Thomas, 52064 Aachen (DE); WEIDNER, Carola, 52064 Aachen (DE)
(74) Representative: Remus, Alvaro Johannes
(86) International application number: PCT/EP2014/059784
(87) International publication number: WO 2014/184199

(56) References cited:
- WO-A1-2013/022872
- WO-A2-2005/085471
- WO-A2-2012/078288
- KR-A- 20110 042 607
- US-A1- 2009 119 022
- US-A1- 2012 251 619
- Anonymous: "Moalic-PERV_A14220-235-Avr_Nhe-HEK_293-D2 Moalic_PERV_A14220_S1_D2_Avr - GSS - NCBI", , 29 August 2008 (2008-08-29), XP055139769, Retrieved from the Internet: URL:http://www.ncbi.nlm.nih.gov/nucgss/FI1 85621 [retrieved on 2014-09-11]
- Anonymous: "454GmaGlobSeed657678 Soybean Seeds Containing Globular-Stage Embryos G - EST - NCBI", , 2 October 2008 (2008-10-02), XP055139763, Retrieved from the Internet: URL:http://www.ncbi.nlm.nih.gov/nucest/GD9 15029 [retrieved on 2014-09-11]
- Anonymous: "101302_1948_2984 Arabidopsis ovule high throughput cDNA library Arabid - EST - NCBI", , 30 December 2007 (2007-12-30), XP055139765, Retrieved from the Internet: URL:http://www.ncbi.nlm.nih.gov/nucest/ES0 71435 [retrieved on 2014-09-11]
- E JOST ET AL: "Epimutations mimic genomic mutations of DNMT3A in acute myeloid leukemia", LEUKEMIA, vol. 28, no. 6, 27 November 2013 (2013-11-27), pages 1227-1234, XP055139632, ISSN: 0887-6924, DOI: 10.1038/leu.2013.362
- XIE S ET AL: "Cloning, expression and chromosome locations of the human DNMT3 gene family", GENE, ELSEVIER, AMSTERDAM, NL, vol. 236, no. 1, 5 August 1999 (1999-08-05), pages 87-95, XP004175451, ISSN: 0378-1119, DOI: 10.1016/S0378-1119(99)00252-8
- C. HOLZ-SCHIETINGER ET AL: "Mutations in DNA Methyltransferase (DNMT3A) Observed in Acute Myeloid Leukemia Patients Disrupt Processive Methylation", JOURNAL OF BIOLOGICAL CHEMISTRY, vol. 287, no. 37, 7 September 2012 (2012-09-07), pages 30941-30951, XP055139627, ISSN: 0021-9258, DOI: 10.1074/jbc.M112.366625
- MELKI JOHN R ET AL: "Concurrent DNA hypermethylation of multiple genes in acute myeloid leukemia", CANCER RESEARCH, AMERICAN ASSOCIATION FOR CANCER RESEARCH, US, vol. 59, no. 15, 1 August 1999 (1999-08-01), pages 3730-3740, XP002196183, ISSN: 0008-5472

## Description

### Background of the invention

Basically, the invention relates to a method for determining a human individual's predisposition to contract a hematopoietic malignant myeloid disease. The invention also concerns a method for evaluating a human individual's response to a therapy for treating a hematopoietic malignant myeloid disease.

### Prior art

DNA-methylation (DNAm) of CpG dinucleotides is a key epigenetic process. Upon cell division, the DNAm pattern is maintained on the newly synthesized DNA strand particularly by DNA methytransferase 1 (DNMT1), whereas DNMT3A and DNMT3B act as *de novo* methyltransferases. In addition, a co-regulatory methyltransferase-like protein, DNMT3L, modulates activity and targeting of DNMT3A and DNMT3B. On the other hand, active demethylation is promoted by methyl-CpG binding proteins or *via* a hydroxymethylate intermediate step. Structurally, DNMT1, -3A, and -3B comprise two domains: the highly conserved C-terminal region containing catalytic motifs and the less conserved N-terminal regulatory domains that, in the case of DNMT3A and DNMT3B (as well as DNMT3L), include a PWWP domain (Pro-Trp-Trp-Pro motif) and a PHD finger-like domain (plant homeodomain). These two domains are required for targeting DNMT3s to heterochromatin and contribute to protein-protein interactions (such as recognition of unmethylated histone 3 lysine 4 (H3K4) or interaction with the H3K9 trimethylase SETDB1).

DNAm plays a central role in normal hematopoietic development and there is a growing perception that it is also crucial for the dysregulation in myeloid neoplasias. Conditional ablation of DNMT3A in mice has been shown to increase the stem cell pool and to impair differentiation ¹. The relevance of DNMT3A is further supported by frequent mutations in acute myeloid leukemia (AML) ^{2;3} and myelodysplastic syndromes (MDS) ⁴. About 22% of AML patients harbor mutations in DNMT3A - either at the highly recurrent position R882 ⁵, or at other sites within the gene ². Yan et al have recently reported that mutations in *DNMT3A* are associated alterations of DNAm and/or gene expression profiles (such as HOXB genes) ⁶. Furthermore, mutations in DNMT3A are associated with poor prognosis in AML and have hence been suggested for risk stratification ⁷. Recently, high expression of DNMT3B has also been shown to be a poor prognostic marker in AML ⁸. Furthermore, the role of DNMT-Inhibitors in the treatment of MDS and AML has been addressed in multiple clinical trials: treatment with azacytidine resulted in a longer overall survival, a lower transfusion frequency and a later transformation of MDS into AML. However, the exact mode of action of DNMT-inhibitors, predictive biomarkers, best schedule of administration and combination with other treatment modalities are still unknown.

Interestingly, all three catalytically active DNMTs are subject to extensive tissue- or developmental stage specific alternative splicing. Particularly for DNMT3B, more than 40 different isoforms have been described which are generated by alternative splicing and/or alternative promoter usage. Expression of specific DNMT3B transcripts (including catalytically inactive isoforms) were demonstrated in small non-lung cell cancer, cancer cell lines and primary acute leukemias indicating that these variants may be relevant for development of malignant diseases. Different variants of DNMT3s - with different interaction and binding properties - are even co-expressed in the same cell. Although it has been recognized that alternative isoforms of DNMT3s may be relevant in AML this has hardly been systematically addressed. Particularly little is known how DNAm is affected within the genes *DNMT3A* and *DNMT3B* - such modifications may be involved in the regulation of their alternative isoforms.

Accordingly, the *de novo* methyltransferases DNMT3A and DNMT3B play a pivotal role in epigenetic regulation and development of malignant diseases such as AML. However, little is known how these genes themselves are epigenetically controlled and which role the various transcripts play in this process.

WO 2012/078288 A2 discloses methods for determining the risk of adverse outcome in a subject diagnosed with acute myeloid leukemia (AML) or myelodysplastic syndrome (MDS). The methods comprise providing a sample comprising bone marrow cells and/or peripheral blood from a subject diagnosed with AML or MDS, sequencing at least one exon of DNMT3A comprised by the sample, and identifying mutations, if any, in the DNMT3A gene.

### Summary of the invention

It is the object of the invention to provide methods for use in diagnostics, approaches and selection of therapeutic regimen. The object is achieved by providing a method for determining a human individual's predisposition to contract a hematopoietic malignant myeloid disease, wherein the myeloid disease is acute myeloid leukemia (AML) or myelodysplastic syndrome (MDS), comprising:
Providing at least one nucleic acid of the human individual;
Determining a methylation level of at least one specific region of the nucleic acid comprising at least one CpG-dinucleotide, wherein the specific region consists of the gene of the DNA-methyltransferase DNMT3A; and
Comparing the determined methylation level with at least one reference methylation level of the specific region(s), wherein the risk of the human individual to contract the myeloid disease is increased if the determined methylation level is increased compared to the reference methylation level.

The reference methylation level may be an empirically determined methylation level of the specific region of healthy individuals and/or an average methylation level of the specific region of healthy individuals. That is, the reference value may be determined by way of an empirical study with healthy individuals, i.e. persons that do neither exhibit the disease nor belong to a risk-group. In order to achieve a reliable value for the percentage of methylated CpG-dinucleotides of a given CpG-dinucleotide site, an average methylation level can be determined to define the reference value.

The object is further achieved by providing a method for evaluating a human individual's response to a therapy for treating a hematopoietic malignant myeloid disease, wherein the myeloid disease is acute myeloid leukemia (AML) or myelodysplastic syndrome (MDS), and wherein the therapy has been performed by administering at least one DNA-methyltransferase inhibitor or at least one anthracycline antibiotic, comprising:
Providing at least one first nucleic acid of the human individual;
Determining a first methylation level of at least one specific region of the first nucleic acid comprising at least one specific CpG-dinucleotide, wherein the specific region consists of the gene of the DNA-methyltransferase DNMT3A;
Providing at least one second nucleic acid of the human individual after the therapy was performed;
Determining a second methylation level of the specific region(s) of the second nucleic acid, wherein the second methylation level is a methylation level of the same specific CpG-dinucleotide; and
Comparing the first methylation level with the second methylation level, wherein the therapy has a positive effect on the myeloid disease if the second methylation level is decreased compared to the first methylation level.

In this method, the first methylation level is a methylation level of a specific CpG-dinucleotide before the therapy is performed, while the second methylation level is a methylation level of the same specific CpG-dinucleotide after the therapy was performed.

In one embodiment of the invention, the DNA-methyltransferase inhibitor is azacitidine or decitabine. The anthracycline antibiotic may be daunorubicine or idarubicine.

In another preferred embodiment of the invention, the specific region comprises at least one promoter region.

In another preferred embodiment of the invention, the specific region comprises the nucleotide sequence of SEQ ID NO: 1.

### SEQ ID NO: 1

Part of the nucleotide sequence (DNA) of the DNMT3a gene. CpG-dinucleotides sites presented by the InfiniumHumanMethylation 450k BeadChip (Illumina, San Diego, USA) are underlined and printed in bold letters:

DNMT3A and DNMT3B are de novo DNA-methyltransferases which play pivotal roles in development of malignant diseases such as acute myeloid leukemia (AML). Both of them are alternatively spliced in a tissue- and disease-specific manner. However, so far little is known about the functional role of specific splice variants and their impact on the DNA methylation (DNAm) pattern. With the present invention it was discovered that an internal promoter region of DNMT3A is significantly hypermethylated in about 40% of AML patients (P < 10⁻⁷) which is associated with down-regulation of specific DNMT3A transcripts.

According to the invention, the determined methylation level is increased compared to the reference methylation level, wherein the increased methylation level indicates an increased risk of the human individual to contract the malignant disease. In particular in respect of AML, the determined methylation level is 10 % or higher. That is, the risk of a human individual to contract acute myeloid leukemia is increased if the determined methylation level is 10% or higher. For example, an increased methylation level in the sense of the present invention can be in the range of 10 % to 50 %, 10 % to 45 %, 10 % to 40 %, 10 % to 35 %, 10% to 30 %, 10 % to 25 %, 15 % to 45 %, 20 % to 40 %, 25 % to 35 %, 20 % to 50 %, or 30 % to 50 %.

In another preferred embodiment of the invention, the DNA is derived from blood cells selected from the group consisting of monocytes, T cells, hematopoietic progenitor cells (HPCs), lymphocytes, mononuclear cells (MNCs), and leucocytes.

In one embodiment of the invention, the blood cells are provided in the form of at least one peripheral blood sample of the human individual.

Two assay designs for pyrosequencing using the following primers may be used to analyze DNAm in the specific region:

| | | |
|---|---|---|
| 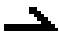 PCR | F1 | GGTTTGGGTTTATTGTAGGAAGGTTATTAAGGT (SEQ ID NO: 3) |
| 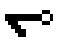 PCR | R1 | AATCCAAAACCCCCCTATCACGAAA (SEQ ID NO: 4) |
| ^{→}Sequencing | S1 | GTGGGGGGAGATAAA (SEQ ID NO: 5) |

| | | |
|---|---|---|
| 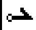 PCR | F3 | TTAGGTTCGGGAGTATTAGGGGGAGG (SEQ ID NO: 6) |
| 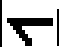 PCR | R3 | AACAAAACCAAACAAAATAAATAAAACAACCC (SEQ ID NO: 7) |
| ^{←}Sequencing | S3 | AAATAAAACAACCCTTAAAA (SEQ ID NO: 8) |

According to the invention, the methylation level of the specific region may be determined, for example, by methylation specific PCR, sequence analysis of bisulfite-treated DNA, CHIP-sequencing (Illumina Human Methylation BeadChip Technology), Methyl-CAP-sequencing, Next-Generation-Sequencing, COBRA-Assay and methylation-specific restriction patterns. Alternatively, it is possible to determine the methylation level by MassARRAY assay. It is preferred that the methylation status is determined by pyrosequencing of bisulfite-treated DNA as described in more detail herein below, and which allows identifying whether a specific CpG-dinucleotide was methylated or not, and thereby provides an accurate value for the percentage of methylated CpG-dinucleotides of a given CpG-dinucleotide site.

According to another or additional embodiment of the invention, methylation of the at least one CpG-dinucleotide is determined by sequence analysis of bisulfite treated DNA. The method of methylation analysis utilizing sequence analysis of bisulfite-treated DNA involves bisulfite treatment of the DNA to be analyzed, PCR amplification of the bisulfite treated DNA, cloning of the amplification product, and standard dideoxynucleotide DNA sequencing of the cloned DNA fragments to directly determine the nucleotides resistant to bisulfite conversion. Preferred primers for PCR amplification are designed to be strand-specific but not methylation-specific, i.e. the nucleotide sequence of the preferred primers do not comprise a sequence corresponding to a nucleotide sequence including a CpG dinucleotide. Hence, the preferred primers for PCR amplification flank but do not involve the methylation site or methylation sites of interest. However, in an additional or alternative embodiment, at least one or both, the forward primer and the reverse primer for PCR amplification may cover one or more CpG-dinucleotides. To be strand specific and to allow amplification of methylated as well as non-methylated DNA fragments, a mixture of primers may be utilized, wherein the mixture of strand primers consists of primers having a pyrimidine nucleotide (Y) for the cytosine of the CpG dinucleotide within the DNA fragment to be amplified and covered by said strand primer, i. e. a C for amplification of said DNA fragment which is methylated at said C, or a T for amplification of said DNA fragment which is not methylated at said C. Therefore, the PCR amplification will amplify both methylated and unmethylated sequences, in contrast to methylation-specific PCR. All sites of unmethylated cytosines are displayed as thymines in the resulting amplified sequence of the sense strand, and as adenines in the amplified antisense strand. This method requires cloning of the PCR products prior to sequencing for adequate sensitivity. Alternatively, nested PCR methods can be used to enhance the product for sequencing. Pyrosequencing may also be used to analyze bisulfite-treated DNA without using methylation-specific PCR. Following PCR amplification of the region of interest, pyrosequencing is used to determine the bisulfite-converted sequence of specific CpG sites in the region. The status of C-to-T at individual sites can be determined quantitatively based on the amount of C and T incorporation during the sequence extension.

Also disclosed therein is a method for determining a human individual's predisposition to contract a malignant disease may be performed in combination with the method according to the invention as described above, said method comprising:
Providing at least one cell of the human individual;
Analyzing the expression and/or sequence of at least one specific transcript of a DNA of the cell; and
Comparing the analyzed expression data and/or transcript sequence with the expression data and/or transcript sequence of normal cells, wherein the risk of the human individual to contract the malignant disease is increased if the analyzed expression data and/or transcript sequence of the human individual's cells is/are altered in respect of the expression data and/or transcript sequence of normal cells.

In this embodiment the analysis comprises the detection of splice variants of the specific transcript.

The invention is further described in detail with reference to the figures.

### Brief description of the figures

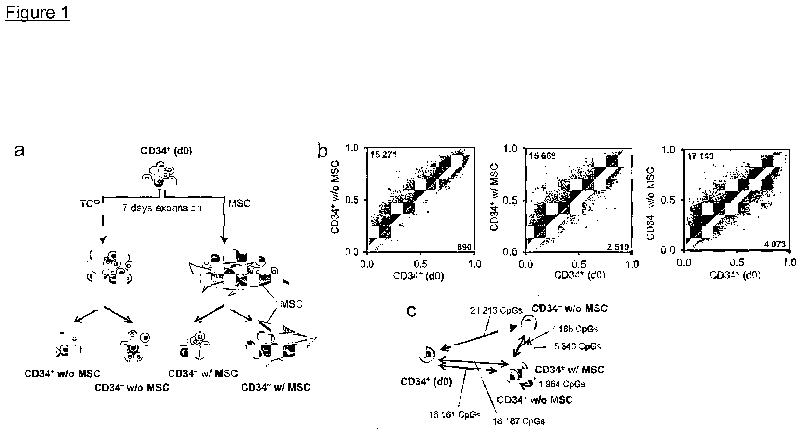

**Figure 1** shows:
   **Culture expansion of CD34⁺ cells entails hypermethylation at specific CpG sites.**
   **(a)** Experimental design to address DNAm upon culture expansion. CD34⁺ cells (d0) were cultured in parallel either with or without mesenchymal stromal cells (MSCs). After one week, HPCs were separated into CD34⁺ and CD34⁻ fractions.
   **(b)** DNAm profiles were subsequently analyzed by Infinium HumanMethylation450k Bead Chips. Scatter plots demonstrate significant DNAm changes upon culture expansion (adjusted P < 0.05; the number of significant CpGs is indicated). In comparison to freshly isolated CD34⁺ (d0) cells, all expanded fractions (CD34⁺ w/o MSC, CD34⁻ w/o MSC or CD34⁺ w/ MSC) revealed highly significant hypermethylation at specific CpG sites. **(c)** Numbers of differentially methylated CpG sites (hyper- and hypomethylated) between the different subsets. Overall, DNAm profiles of CD34⁺ w/o MSC and CD34⁺ w/ MSC were closely related (Weidner et al., Scientific Reports 2013; link: http://www.nature.com/srep/2013/131128/srep03372/full/srep03372.html).

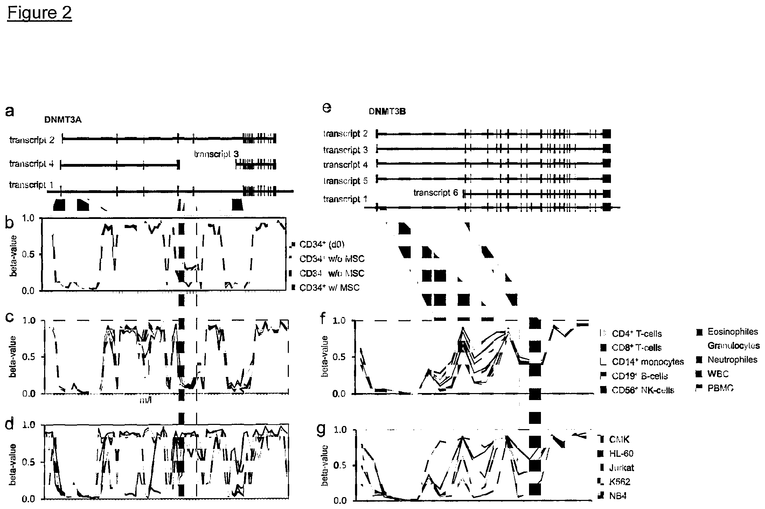

**Figure 2** shows:
   **DNAm and transcripts of *DNMT3A* and *DNMT3B.***
   **(a)** Different transcripts of *DNMT3A* are depicted (http://www.ncbi.nlm.nih.gov/; Gene ID1788). **(b)** DNAm of either freshly isolated CD34⁺ HPCs or expanded CD34⁺ and CD34⁻ subpopulations is presented for CpG sites of *DNMT3A.* Culture-associated hypermethylation is located at an internal promoter region between transcripts 3 and 4 (Weidner et al., Scientific Reports 2013; P < 10⁻⁴⁶). **(c)** This region is hardly methylated in various hematopoietic cell types (GSE35069). However, another region appears to be differentially methylated in myeloid and lymphatic cells (m/l). **(d)** In contrast to primary cells, DNAm profiles of malignant cell lines were highly methylated at the internal promoter region of *DNMT3A* (GSE40699). **(e)** Different transcripts of *DNMT3B* (http://www.ncbi.nlm.nih.gov/; Gene ID1789). This gene did not reveal differential DNAm upon culture expansion of HPCs (data not shown). However, in analogy to DNMT3A there is an internal promoter region before transcript 6 with very little variation in primary cells **(f;** GSE35069) whereas the DNAm level varies extensively in the malignant cell lines **(g;** GSE40699).

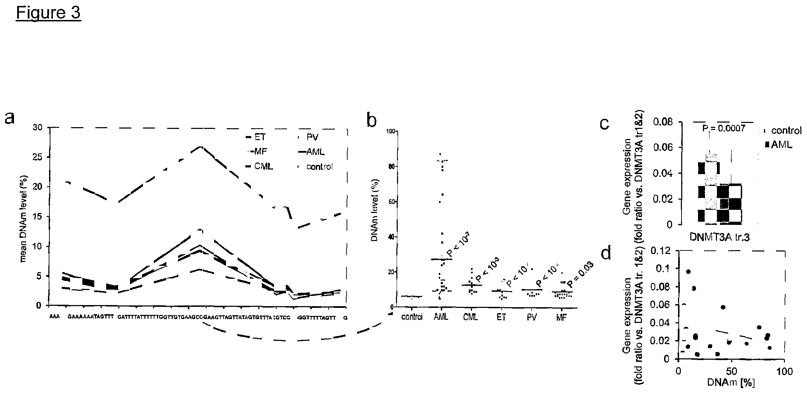

**Figure 3** shows:
   **DNAm within DNMT3A is particularly increased in AML samples.**
   **(a)** DNAm within the internal promoter region of DNMT3A was analyzed by pyrosequencing in 10 healthy controls, 70 AML samples, 15 CML samples, 13 samples of essential thrombocythemia (ET), 14 samples of polycythemia vera (PV), and 20 samples of myelofibrosis (MF). Mean DNAm levels are indicated for six CpG sites. **(b)** Individual DNAm levels are exemplarily depicted for the CpG site indicated by the grey arrow. Overall, DNAm was significantly higher in AML, CML, and MPN samples than in controls. Notably, about 40% of AML samples revealed a very high DNAm level at this CpG site. **(c)** Differential expression of transcripts 1+2, 3, and 4 was analyzed by qRT-PCR in 18 healthy controls and 26 AML samples. In AML samples expression of transcript 3 was significantly downregulated in comparison to transcript 1+2. **(d)** The DNAm level at the relevant CpG site is inversely correlated with expression of transcript 3 (Weidner et al., Scientific Reports 2013; Jost et al., Leukemia 2014).

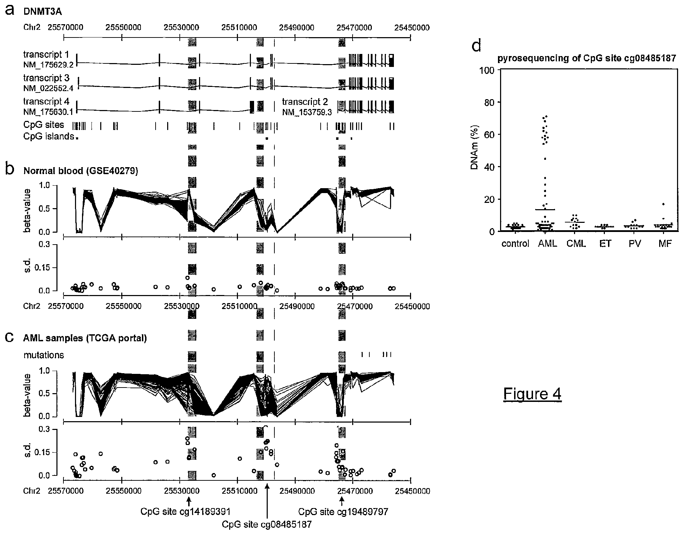

**Figure 4** shows:
   **Variable DNAm within DNMT3A in AML.**
   **(a)** Schematic presentation of four different transcripts for DNMT3A. The positions of corresponding CpG probes represented on the HumanMethylation450K BeadChip platform and their relation to CGls are depicted. **(b)** β-Vaiues (DNAm level) for each of these CpG sites were analyzed in 656 normal blood samples. Standard deviation (s.d.) of β-values revealed relatively little interindividual variation at all CpG sites. **(c)** However, DNAm profiles of 194 AML samples revealed three DMRs that are highlighted in red. Probe set IDs for the most prominent CpG sites are indicated by arrows. Furthermore, locations of genomic mutations are indicated. Notably, all mutations in DNMT3A are related to exons of transcript 2. (d) DNAm at the CpG site cg08485187 (within DMR2) was subsequently analyzed by bisulfite pyrosequencing in peripheral blood of healthy controls and patients with AML, CML, essential thrombocythemia (ET), polycythemia vera (PV) and myelofibrosis (MF).

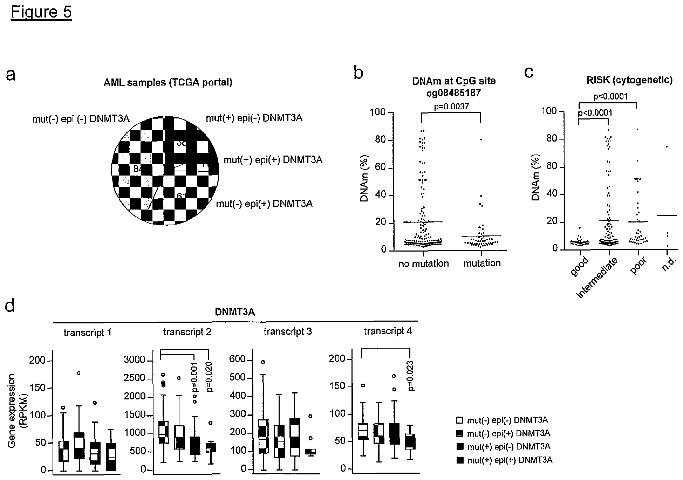

**Figure 5** shows:
   **Interplay of epimutations and mutations in DNMT3A.**
   **(a)** DNAm profiles of TCGA9 were categorized in samples with DNMT3A mutation (mut+) and those with aberrant DNA hypermethylation at cg08485187 (β-value >0.1), which has been classified as epimutation (epi+). **(b and c)** Aberrant DNA hypermethylation was significantly lower in AML samples with DNMT3A mutations and it was hardly associated with a good cytogenetic risk score (ND=not determined). **(d)** Analysis of RNA-sequencing data9 revealed that DNMT3A transcript 2 is slightly less expressed in samples with epimutation and significantly downregulated in samples with DNMT3A mutations. Statistical significance was estimated by Wilcoxon's rank-sum test. RPKM=reads per kilobase per million reads.

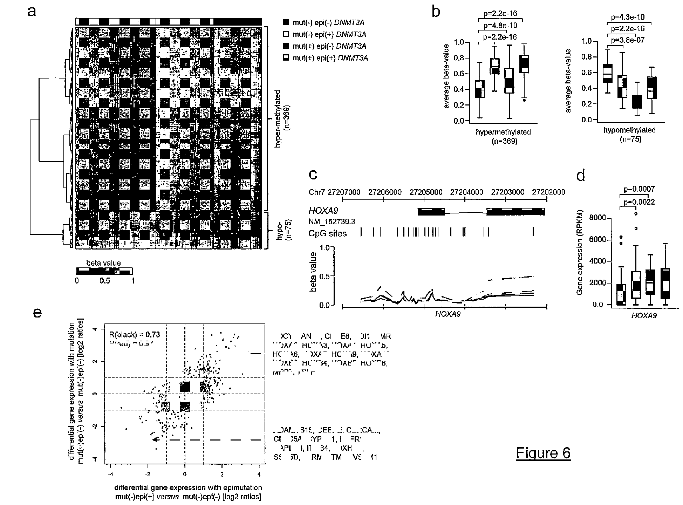

**Figure 6** shows:
   **DNAm changes in samples with epimutation/mutation in DNMT3A.**
   **(a)** Heatmap of 444 CpGs with significant DNAm changes between samples with epimutation and/or mutation in comparison with AML samples without such DNMT3A modifications (adjusted P-value <0.05; differential DNAm >20%). In particular, the DNAm pattern of hypomethylated CpGs was related in samples with either epimutation or mutation. **(b)** Average DNAm levels (β-values) of these CpG sites that are either hypermethylated or hypomethylated. **(c)** DNAm pattern of CpG sites related to the gene HOXA9 is exemplarily depicted: AML samples with either mutations or epimutations in DNMT3A revealed significantly lower DNAm levels.
   **(d)** Conversely, HOXA9 is significantly higher expressed in samples with DNMT3A mutation or epimutation. Statistical significance was estimated by Wilcoxon's rank-sum test. **(e)** Differential gene expression in AML samples with or without epimutation was plotted against differential gene expression in AML samples with or without genomic mutation in DNMT3A. Genes with differential expression in either one or both comparisons are highlighted in black and red, respectively. Gene IDs are provided for genes significant in both comparisons.

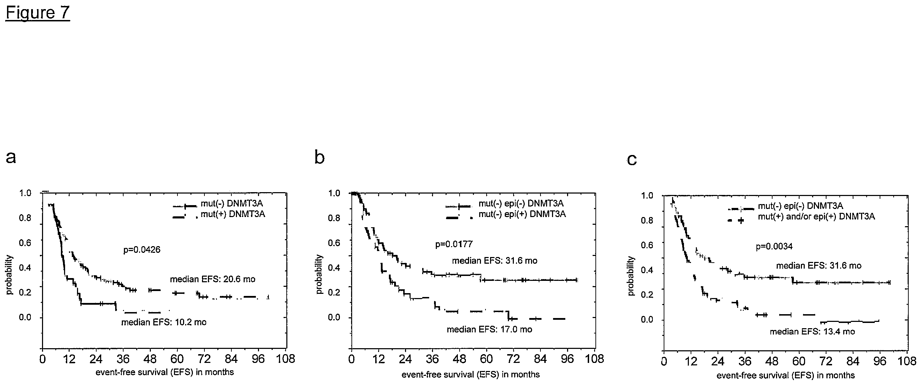

**Figure 7** shows:
   **Kaplan-Meier analysis of event-free survival (EFS) of individuals with AML.**
   **(a)** Patients with DNMT3A mutations (mut+) reveal a significantly shorter EFS than patients without mutation (mut-) in AML patients from TCGA (n=194). **(b)** In analogy, patients with epimutation (epi+) revealed a significantly shorter EFS than those without epimutation (epi-; samples with DNMT3A mutation were excluded from this analysis; n=145). **(c)** Results became even more significant when patients with normal DNMT3A status (mut- and epi-) were compared with those with mutation and/or epimutation (n=194).

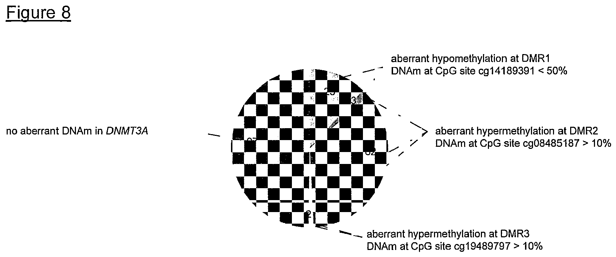

**Figure 8** shows:
   **A pie diagram for the interplay of three DMRs in DNMT3A.**
   DNA-methylation profiles of AML samples from the TCGA repository data³ revealed three differentially methylated regions (DMRs). Based on DNAm in normal blood⁹ we defined cutoffs for aberrant DNAm: DMR1, particularly at CpG site cg14189391, was always methylated more than 50% in normal blood,⁹ whereas it was hypomethylated (DNAm < 50%) in 26 AML samples. In contrast DMR2 and DMR3 were non-methylated in normal blood⁹ (particularly at CpG sites cg08485187 and cg 19489797), whereas they were hypermethylated (DNAm > 10%) in 72 and 9 AML samples, respectively. Notably, overlapping hypomethylation at DMR1 and hypermethylation at DMRs 2 or 3 was underrepresented (p = 0.0038 and p = 0.61, respectively; two-sided Fisher's exact test), whereas hypermethylation correlated at DMR2 and DMR3 (p = 0.013).

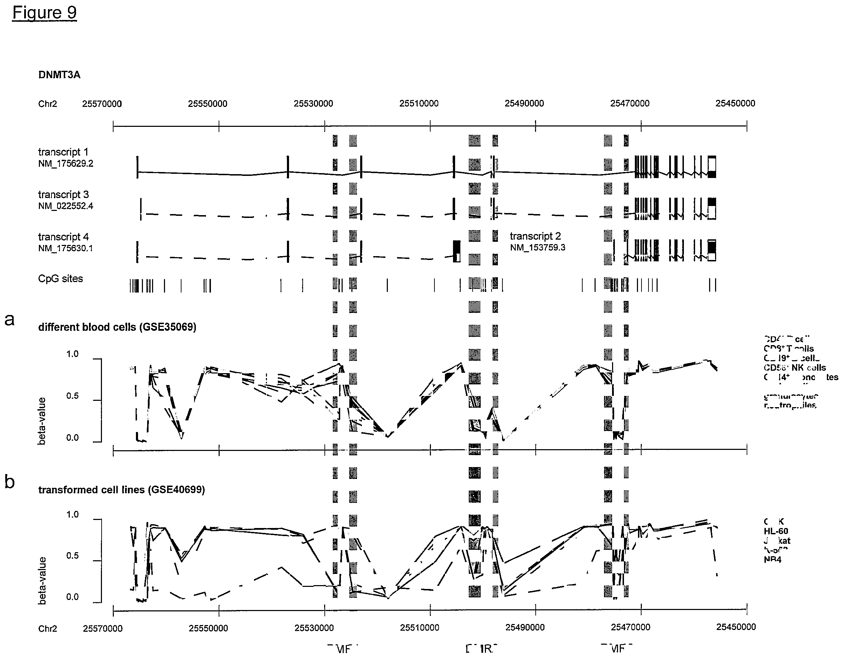

**Figure 9** shows:
   **DMRs of DNMT3A in hematopoietic cell types and malignant cell lines.**
   **(a)** Beta-values of CpG sites within DNMT3A were analyzed in DNAm profiles of various different cell types (Th cells = CD4+ T cells, Tc cells = CD8+ T cells, NK cells = CD56+ NK cells, B cells = CD19+ B cells, monocytes = CD14+ monocytes, eosinophiles, granulophiles, and neutrophiles).¹⁰ DNAm at DMR1 revealed some variation between different cell types and it appeared to be hypomethylated particularly in myeloid cells - with may coincide with hypomethylation in AML. However, DMR2 and DMR3 were rather non-methylated irrespective of the corresponding cell type. **(b)** In contrast, DNAm profiles of malignant cell lines (GSE40699, generated by ENCODE) revealed aberrant hypermethylation particularly at DMR2.

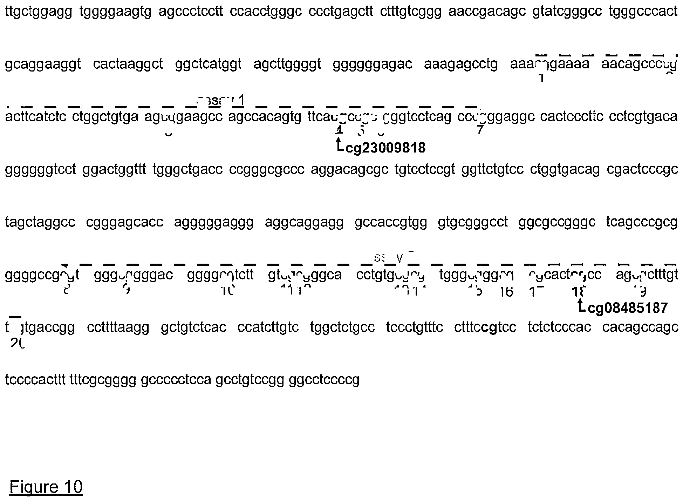

**Figure 10** shows:
   **Bisulfite pyrosequencing assays of DMR2.**
   The nucleotide sequence shown is SEQ ID NO: 1. DNAm was analyzed in blood samples using two different pyrosequencing assays. CpG sites which are covered by these assays are enumerated by numbers 1 to 20. These assays comprise two CpG sites which are also addressed by probe sets of the Illumina HumanMethylation450k BeadChip (cg23009818 and cg08485187).

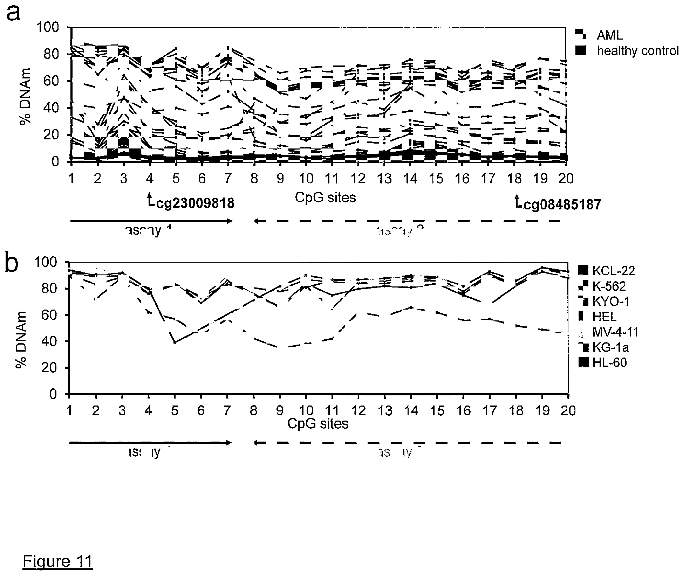

**Figure 11** shows:
   **Bisulfite pyrosequencing results of individual CpG sites.**
   **(a)** Blood from healthy controls (n = 26) and from AML patients (n = 88) were analyzed with the two pyrosequencing assays. DNAm was always below 10% in all healthy controls and at all CpG sites. DNA-hypermethylation in AML samples was not restricted to individual CpGs but appeared to be relatively constant throughout the whole DMR. **(b)** Hypermethylation was also observed in various cell lines derived from AML (KCL-22, K-562, KYO-1, HEL, MV-4-11, KG-1a, and HL-60).

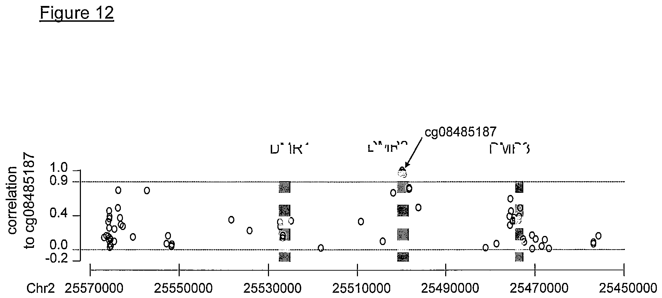

**Figure 12** shows:
   **Correlation of DNAm within the CpG island of DMR2.**
   Besides the CpG site cg08485187, three other probe sets were located in the same CGI of DMR2. All of them showed very high correlation coefficients with DNAm at cg08485187 in TCGA data (cg23009818: R=0.976; cg13558695: R=0.977; cg08493294: R=0.965). In addition, cg17742416, which is in the shore region, is also highly correlated (R=0.948). DNAm of all 20 CpGs covered by the pyrosequencing assays revealed a correlation of more than 0.95, too (data not shown). Therefore, DNAm at cg08485187 is a good surrogate for overall methylation of the CpG island (CpG:_61) in DMR2.

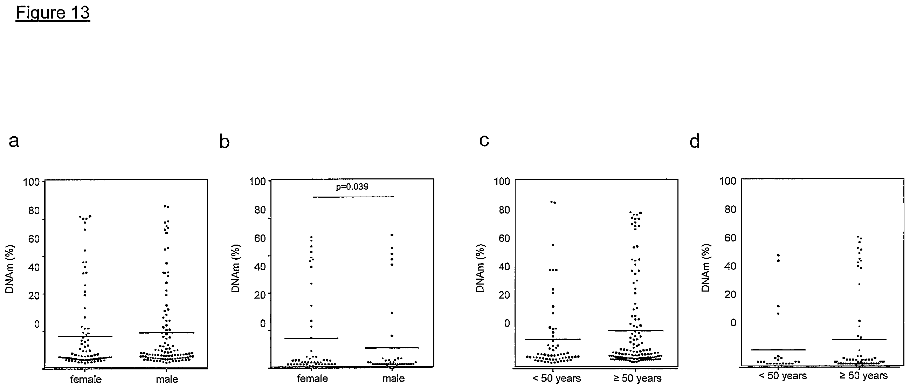

**Figure 13** shows:
   **Association of DNAm at DMR2 with gender and age.**
   DNAm values at cg08485187 was analyzed in TCGA repository data³ **(a, c)** and in our pyrosequencing data **(b, d).** There was not clear association with gender in TCGA data **(a)** and it was only slightly increased in our female samples **(b).** In tendency, DNAm at DMR2 was higher in samples of elderly donors but this was not significant **(c, d).** Statistical significance was estimated by Wilcoxon rank-sum test.

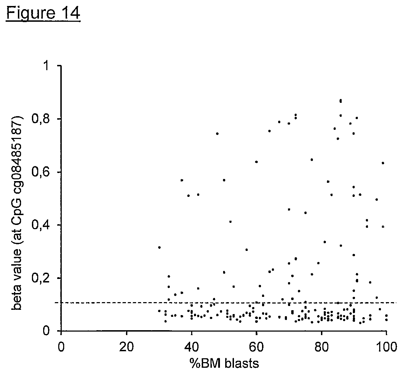

**Figure 14** shows:
   **DNAm in relation to blast counts.**
   **(a)** The DNA methylation level at the relevant CpG site (cg08485187) is plotted against blast counts of the TCGA repository data.³ The dotted line indicates 10% DNAm cutoff which has been used to discern samples with epimutation. There is no linear relationship between aberrant DNAm and blast counts. The high DNAm levels in several samples indicate that the epimutation affects both alleles. **(b)** For additional control, we have analyzed DNAm by pyrosequencing in four stem cell harvests obtained by leukapheresis. Despite high content of stem and progenitor cells the DNAm level was always low.

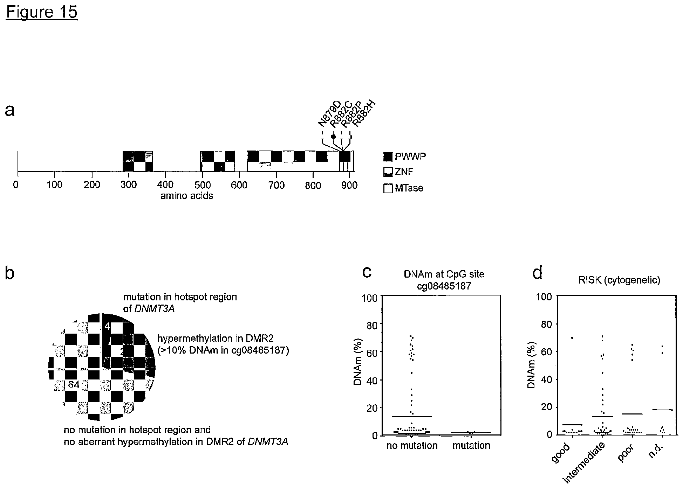

**Figure 15** shows:
   **Mutational analysis of the DNMT3A hotspot region.**
   **(a)** Schematic presentation of DNMT3A. The conserved proline-tryptophan-tryptophan-proline (PWWP), zinc finger (ZNF), and methyltransferase (MTase) domains are shown. In our exploratory analysis we sequenced exons 18, 19, and 23. These assays account for 70% of the genomic mutations identified in TCGA repository data. In three AML patients, we observed mutations at the hotspot region (R882), one sample revealed a so far non-described mutation in close vicinity (N879), and one patient revealed a stop mutation in exon 18 (C710). Sequencing of HL-60, HEL, KG-1a, MV-4-11, NALM6, K-562, and KCL-22 cell lines did not reveal mutations at that region (data not shown). DNAm was only analyzed in DMR2 by bisulfite pyrosequencing. **(b)** Pie diagram of AML patients with mutation and epimutation (DNAm level at cg08485187 > 10% as determined by bisulfite pyrosequencing). **(c, d)** Epimutations were only found in samples without mutation at the frequently mutated regions (not significant; p = 0.58, Fisher's exact test) and they were hardly associated with good cytogenetic risk score (not significant; p = 0.29; n.d. = not determined).

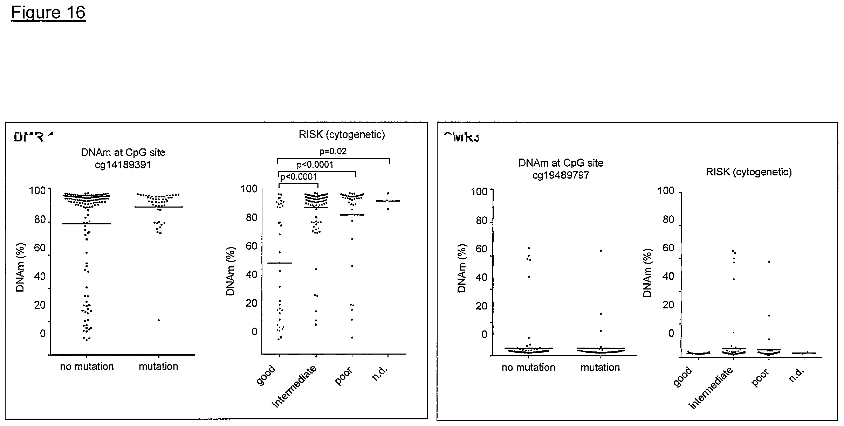

**Figure 16** shows:
   **Hypomethylation at DMR1 is associated with good cytogenetic risk.**
   Analysis of DNAm profiles of AML samples in conjunction with mutation status of DNMT3A and cytogenetic risk score³ revealed that aberrant hypomethylation of DMR1 was rather observed in AML without mutation in DNMT3A - in analogy to aberrant hypermethylation of DMR2. However, DNAm at DMR1 was significantly lower in samples with good cytogenetic risk score whereas hypermethylation in DMR3 occurred rather in AML with intermediate or poor risk (n.d. = not determined).

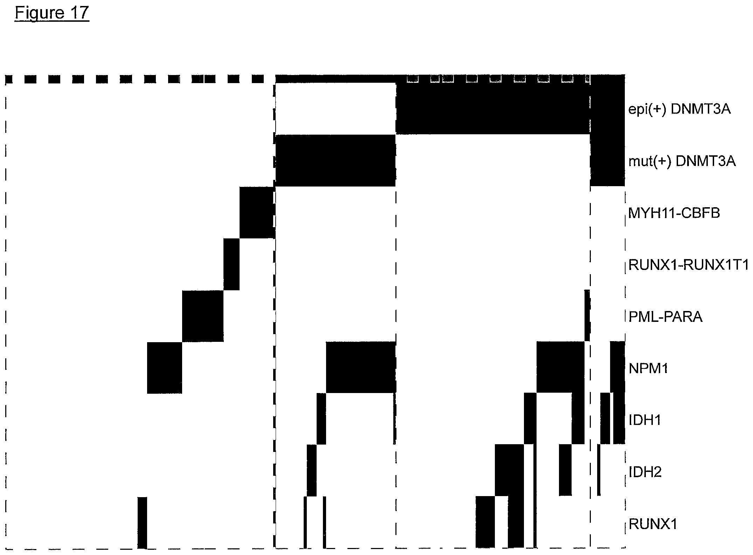

**Figure 17** shows:
   **Organization of mutations in DNMT3A categories.**
   AML samples of TCGA have been analyzed using either whole-genome sequencing (50 cases) or whole-exome sequencing (150 cases).³ 194 samples with DNAm profiles were subsequently grouped in those with epimutation (epi+) and/or mutation (mut+) in DNMT3A and analyzed with regard to gene fusions or mutations. A selection of relevant mutations is presented which revealed differences in samples with DNMT3A modifications (black depicts mutation or epimutation of corresponding genes). For clarity, other mutations are not presented. Notably, MYH11-CBFB, RUNX1-RUNX1T1 and PML-PARA fusions were significantly enriched in epi(-)mut(-) samples (p < 0.001, p = 0.009, and p < 0.001, respectively; chi-squared test) - these fusions have been associated with good prognosis.¹¹ In contrast, the frequent mutations in NPM1, IDH1, IDH2 and RUNX1 were significantly enriched in patients with mutations and/or epimutation in DNMT3A (p < 0.001, p < 0.001, p < 0.001, and p < 0.027, respectively; the color code on the left corresponds to DNMT3A modifications according to Figure 2A).

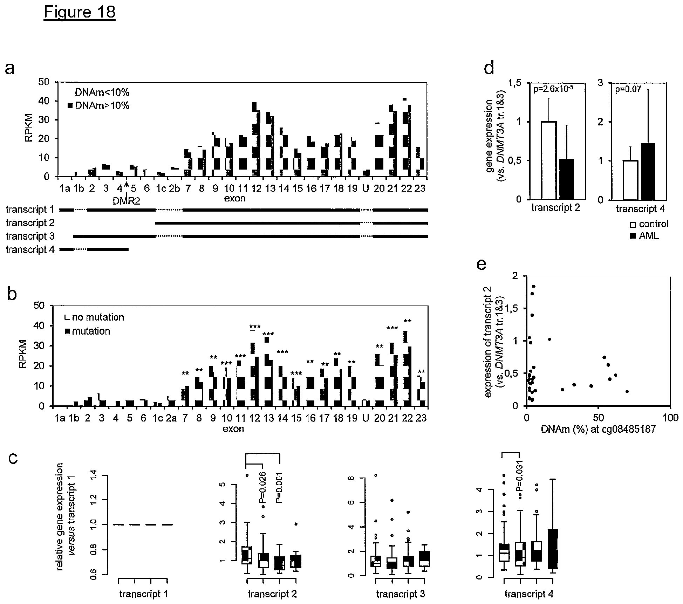

**Figure 18** shows:
   **Expression of DNMT3A transcripts.**
   **(a)** Transcription of exons were analyzed in RNA-sequencing data of TCGA.³ The scheme depicts which exons are comprised in the four main transcripts of DNMT3A (alternative exons included in variant transcripts are indicated by letters; U = undefined transcript association). In samples with aberrant hypermethylation in DMR2 (DNAm > 10% at cg08485187) particularly exons involved in transcript 2 were slightly less expressed than in patients without epimutation (RPKM = reads per kilobase per million reads). The same tendency was also observed in microarray data (data not shown).³ **(b)** Notably, these exons revealed an even more pronounced down-regulation in samples with DNMT3A mutation (** p < 0.01; *** p < 0.001; two-sided t test). **(c)** To further analyze if the epimutation and mutation have impact on the relative expression of variant transcripts we used quantile normalization to scale the expression levels of four transcripts to same level. Expression levels of individual transcripts were then normalized to transcript 1. The analysis revealed that transcript 2 and transcript 4 are relatively less expressed in AML with epimutation in DNMT3A (Wilcoxson rank-sum test). **(d)** qRT-PCR analysis of variant transcripts in our AML samples was hampered by the high variation of housekeeping genes (e.g. GAPDH) between AML samples (n = 18) and healthy controls (n = 34). Therefore, we normalized towards expression levels of transcript 1&3 (primer pairs do not discern between transcript 1 and 3). Overall, transcript 2 of DNMT3A appeared to be less expressed in AML samples than in healthy controls. In contrast, transcript 4 appeared to be slightly increased in AML samples. **(e)** High levels of DNAm at DMR2 were rather associated with low expression of transcript 2. This trend is in accordance with TCGA data, but it did not reach significance level.

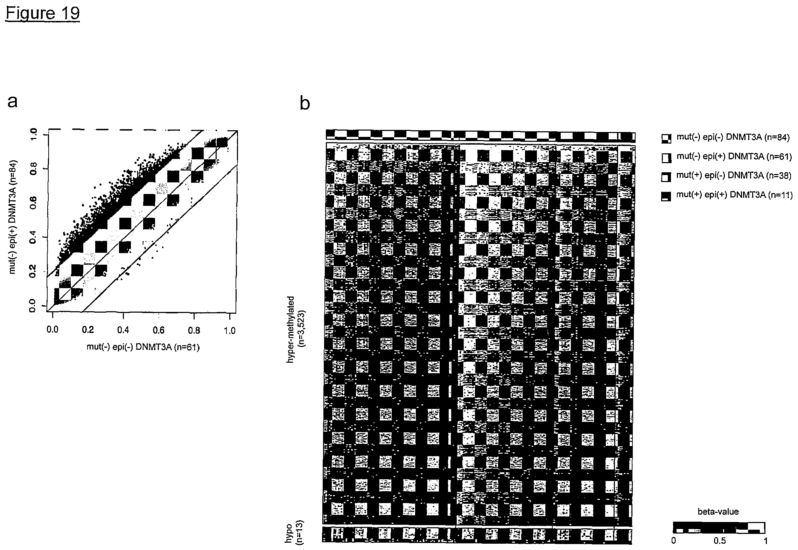

**Figure 19** shows:
   **DNAm changes in AML with epimutation in DNMT3A.**
   DNAm profiles of TCGA³ were compared between samples with or without epimutation (mutated samples were excluded from this analysis; mut-). **(a)** Scatter-plot analysis reveals that epimutation (epi+) is associated with significant hypermethylation of many CpGs (adjusted p-value < 0.05; DNAm changes > 20%; depicted in black). **(b)** Heat-map presentation of the corresponding 3,536 CpGs. Notably, the few hypomethylated CpGs revealed a similar pattern also in samples with DNMT3A mutations.

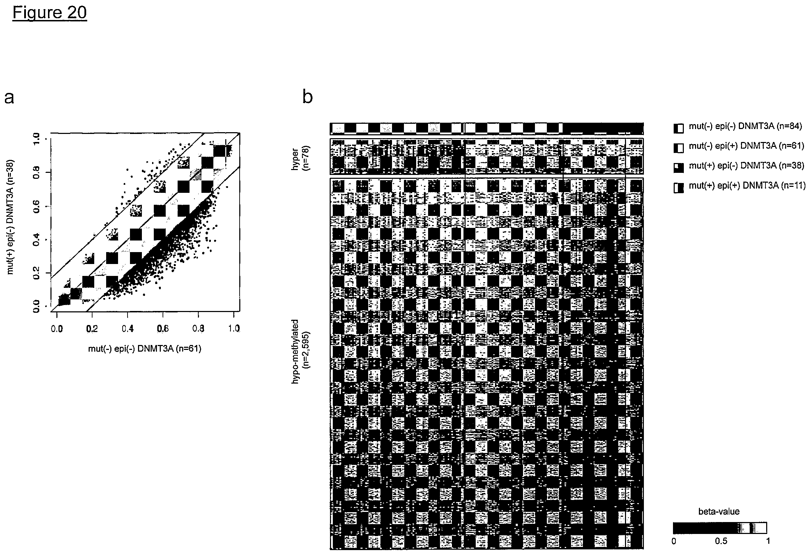

**Figure 20** shows:
   **DNAm changes in AML with mutation in DNMT3A.**
   DNAm profiles of TCGA³ were compared between samples with or without mutation (mut+/-). Samples with epimutation were excluded from this analysis (epi-). **(a)** The scatter-plot reveals that mutations in DNMT3A are associated with significant hypomethylation of many CpGs (adjusted p-value < 0.05; DNAm changes > 20%; depicted in black). **(b)** Heat-map presentation of the corresponding 2,673 CpGs. Notably, the few hypermethylated CpGs revealed a similar pattern also in samples with DNMT3A epimutations.

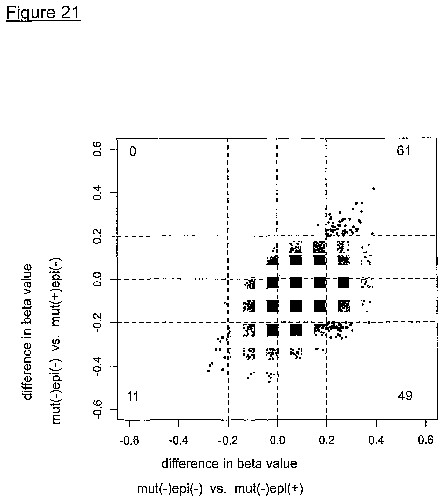

**Figure 21** shows:
   **Correlation of differential DNAm in patients with either epimutation or mutation.**
   Differential DNAm levels in patients with or without epimutation (mut-epi- versus mut-epi+) were plotted against differential DNAm levels in patients with or without mutation (mut-epi- versus mut+epi-). CpG sites with differential DNAm of more than 20% in both comparisons are depicted in black. Chi-square analysis of these CpG sites indicated that there is a significant relationship between the two comparisons (p = 0.0005).

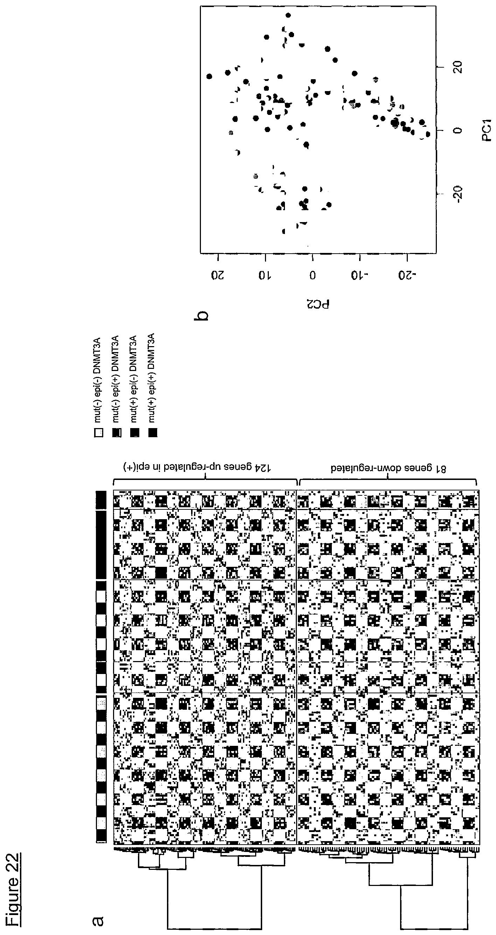

**Figure 22** shows:
   **Gene expression in AML with epimutation in DNMT3A.**
   **(a)** RNA-sequencing data from TCGA³ were compared in samples with or without epimutation (mutated samples were excluded from this analysis). The heatmap depicts 205 differentially expressed genes. **(b)** PCA analysis of these genes demonstrated that AML samples with mutation in DNMT3A were rather related to samples with epimutation in DNMT3A (PC1 and PC2 = principal component 1 and 2).

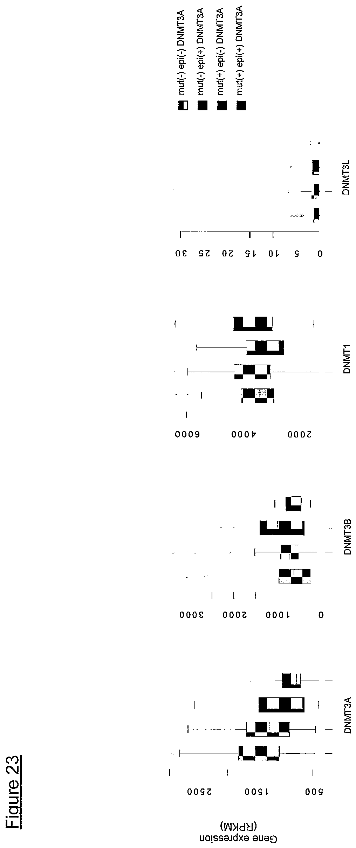

**Figure 23** shows:
   **Gene expression of DNMT1, DNMT3B and DNMT3L is not affected.**
   Given that AML samples with epimutations revealed extensive DNA-hypermethylation, it might be anticipated that gene expression of other methyltransferases has compensatory effects. However, this was not observed in RNA-sequencing data from TCGA³ (results were not significant).

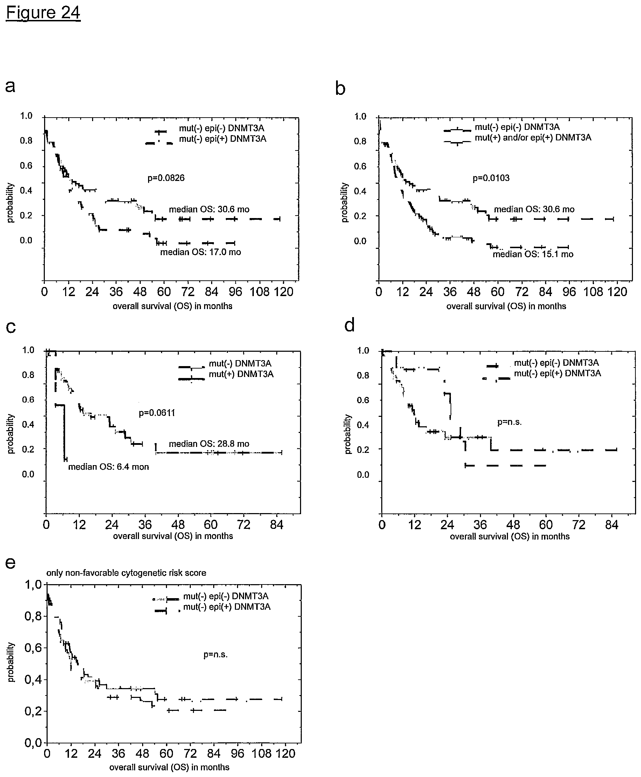

**Figure 24** shows:
   **Overall survival among AML patients with DNMT3A epimutation.**
   **(a)** Patients with DNMT3A epimutations (epi+) reveal shorter OS than patients without epimutation (epi-; samples with mutation were excluded from this analysis) in data from TCGA³ although results did not reach statistical significance (n = 145). **(b)** Comparison of patients with mutation and/or epimutation with patients without such DNMT3A modification revealed significant impact on overall survival (n = 194). **(c)** In our dataset of 88 AMLs the OS was shorter in patients with DNMT3A mutation - in analogy to previous publications.^{2;5;7;12} **(d)** A significant effect of the epimutation on OS was not observed in our dataset (n.s. = not significant). **(e)** Multivariate analysis of OS in AML samples of TCGA with either intermediate or poor cytogenetic risk score did not reveal significant effects - therefore, it is yet unclear if the epimutation in DNMT3A is an independent parameter for prognosis in AML.

### Description of exemplary and preferred embodiments of the invention

Cellular differentiation - and hence the regulation of stem cell function - is based on epigenetic changes. *In vitro* culture of hematopoietic stem and progenitor cells (HPCs) is generally associated with loss of stemness which may also be reflected by epigenetic modifications. To address this question, we have recently analyzed DNAm profiles of either freshly isolated CD34⁺ cells or upon expansion with or without MSCs. These studies were performed with the Illumina HumanMethylation450k BeadChip platform covering more than 480,000 unique CpG sites ¹³. Expansion of HPCs either with or without MSCs revealed relatively little impact on DNAm, although proliferation is greatly increased by stromal support. Notably, all cultured HPCs - even those which remained CD34⁺ - acquired extensive DNA-hypermethylation within seven days *in vitro,* particularly in upstream promoter regions, shore-regions of CpG islands and binding sites for PU.1, HOXA5 and RUNX1 (Figure 1).

Our preliminary screen identified an interesting region within the first intron of *DNMT3B* which might be differentially methylated in AML samples (Figure 2). One of the most significant DNAm changes in this microarray analysis upon culture expansion of HPCs was observed in the gene *DNMT3A* (*P* < 10⁻⁴⁶) - particularly at an internal promoter region which did not affect overall gene expression but might influence expression of isoforms (Figure 2 a-d). In fact, when we analyzed expression of different *DNMT3A* transcripts by qRT-PCR, we observed a significant down-regulation of transcripts 3 and 4 upon culture expansion. Transcript 4 does not possess methyltransferase activity, but down-regulation may still be functionally relevant if it contributes to regulative complexes. Subsequently, we analyzed publically available DNAm profiles of *DNMT3s* in other publically available datasets: under physiologic conditions, various different hematopoietic cell types revealed a very low methylation level at the above mentioned internal promoter region (GSE35069)¹⁰. In contrast, malignant hematopoietic cell lines were highly methylated at this location, although the DNAm profile within DNMT3A was similar to primary cells elsewhere (GSE40699; ENCODE project).

Subsequently, we analyzed DNAm and transcripts of DNMT3B. This gene did not reveal differential methylation upon culture expansion of HPCs (not shown). However, it comprises an interesting promoter region, which is hemimethylated in primary cells and differentially methylated in different leukemic cell lines, too (Figure 2 e-g).

These results led us to the assumption, that the DNAm pattern of *DNMT3A* and *DNMT3B* may be indicative for hematopoietic malignancies. We have established a pyrosequencing assay to address DNAm at an internal promoter region of *DNMT3A.* So far, we have analyzed peripheral blood samples of 10 healthy donors, 15 patients with CML, 20 patients with myelofibrosis (MF), 14 patients with polycythemia vera (PV), 13 patients with essential thrombocythemia (ET), and 70 patients with AML. It was revealed that an internal promoter region of *DNMT3A* is significantly higher methylated in AML samples than in healthy controls (Figure 3). Our results clearly demonstrate that the level of DNAm in this region of the DNMT3A gene is indeed significantly elevated in these myeloid neoplasms and was particularly high in about 40% of AML samples - a disease which often reveals missense and frameshift mutations in the methyltransferase domain of DNMT3A (Figure 3)^{2;14}. Furthermore, qRT-PCR analysis demonstrated very low expression of DNMT3A transcript 3 in these AML samples, suggesting that DNAm at the internal promoter down-regulates its expression. These findings may provide new perspectives for diagnostics and risk assessment - and they indicate that specific isoforms of *DNMT3s* may be relevant for initiation and progression of myeloid diseases.

### Example 1

Infinium Methylation Assays (Illumina, Inc., San Diego, USA) are well-suited to quantitatively interrogate methylation sites at single-nucleotide resolution. For example, the HumanMethylation27 BeadChip allows interrogation of 27,578 CpG loci, covering more than 14,000 genes. Alternatively, the HumanMethylation450 BeadChip offers a unique combination of comprehensive, expert-selected coverage of >450,000 methylation sites, high throughput, and low price, making it ideal for screening genome-wide association study (GWAS) populations.

### DNA isolation and bisulfite conversion:

Genomic DNA was isolated from blood cells using the QIAGEN DNA Blood Midi-Kit. DNA quality was assessed with a NanoDrop ND-1000 spectrometer (NanoDrop Technologies, Wilmington, USA) and gel electrophoresis. 600 ng DNA were subsequently bisulfite converted using the EpiTect Bisulfite Kit (Qiagen, Hilden, Germany).

### DNA methylation profiling:

DNA methylation profiles were analyzed using the HumanMethylation27 or HumanMethylation450 Bead Chip (Illumina, San Diego, USA) according to the manufacturer's instructions. InfiniumHumanMethylation450 BeadChips (Illumina, San Diego, USA). This plattform assays more than 480 000 CpG sites at single base resolution (covering 99% of RefSeq genes and 96% of CpG islands)¹³. During hybridization, the DNA molecules anneal to two different bead types with locus-specific DNA oligomers - one corresponds to the methylated (C) and the other to the unmethylated (T) state. Allele-specific primer annealing is followed by single-base extension using DNP- and Biotin-labeled ddNTPs. After extension, the array is fluorescently stained, scanned, and the intensities of the unmethylated and methylated bead types measured. Hybridization and initial data analysis with the BeadStudio Methylation Module were performed at the DKFZ Gene Core Facility in Heidelberg.

### Analysis of DNA-methylation profiles:

Raw data of new datasets and recently published datasets were quantile normalized to minimize chip effects. Principal components analysis (PCA) was calculated with prcomp in R package stats. For selection of relevant CpG sites we used Pavlidis Template Matching performed with the MultiExperiment Viewer (MeV, TM4.6). Templates were specified that corresponded to the chronological age. The dataset was then searched for matches to the template, based on the Pearson Correlation between the template and methylation level values of the data set. For subsequent analysis we have only considered CpG sites with highly significant hyper- or hypo-methylation according to the corresponding templates (P < 10⁻¹¹). Based on this analysis, we have selected two CpG sites -for simplicity they were termed by their corresponding genes: DNMT3A and DNMT3B.

### Pyrosequencing:

Independent blood samples of known donor's age were subsequently bisulfite converted and analyzed by pyrosequencing. Pyrosequencing was performed at Varionostic GmbH (Ulm, Germany). Primers and sequencing primers are shown below. Other primers than those disclosed herein may be used for amplifying and/or sequencing the respective nucleic acids.

### DNMT3A:

Part of the DNA-Sequence of DNMT3a. CpG sites presented by the 450k Methylation array are highlighted and revealed very similar DNAm changes. This region belongs to the above mentioned internal promoter region.
Sequence (SEQ ID NO: 1):

Two assay designs for pyrosequencing have been used to analyze DNAm in this region:

| | | | | | |
|---|---|---|---|---|---|
| 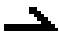 PCR | F1 | GGTTTGGGTTTATTGTAGGAAGGTTATTAAGGT (SEQ ID NO: 3) | 33 | 74.9 | 36.4 |
| 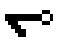 PCR | R1 | AATCCAAAACCCCCCTATCACGAAA (SEQ ID NO: 4) | 25 | 76.8 | 44.0 |
| ^{→}Sequencing | S1 | GTGGGGGGAGATAAA (SEQ ID NO: 5) | 15 | 50.9 | 53.3 |
| Sequence to Analyze | | | | | |

| | | | | | |
|---|---|---|---|---|---|
| 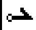 PCR | F3 | TTAGGTTCGGGAGTATTAGGGGGAGG (SEQ ID NO: 6) | 26 | 76.5 | 53.8 |
| 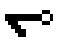 PCR | R3 | AACAAAACCAAACAAAATAAATAAAACAACCC (SEQ ID NO: 7) | 32 | 73.9 | 25.0 |
| ^{←}Sequencing | S3 | AAATAAAACAACCCTTAAAA (SEQ ID NO: 8) | 20 | 50.5 | 20.0 |
| Sequence to Analyze | | | | | |

### DNMT3B

Part of the sequence of DNMT3B. Two CpG sites of which belong to an internal promoter region are highlighted.
Sequence (SEQ ID NO: 2):

### Example 2

### Blood samples:

Blood samples of healthy donors and patients were taken after written consent according to the 'Biobank' rules of the medical faculty of the University of Aachen (Permit Number: EK206/09) and the study has been specifically approved by the local ethics committee. Diagnosis of AML or myeloproliferative neoplasms was established according to the World Health Organization criteria and patient characteristics are summarized in Table 1. For additional control, stem cell harvests obtained by leukapheresis from patients with solid tumors or non-Hodgkin lymphoma without evidence of blood infiltration were analyzed (EK206/09).

### DNAm analysis by pyrosequencing:

Genomic DNA was isolated using the QIAamp DNA Blood Mini Kit (Qiagen, Hilden, Germany). One microgram of DNA was sodium bisulfite modified using the EZ DNA Methylation Kit (Zymo Research, Irvine, CA, USA). The region of interest was amplified by polymerase chain reaction (PCR) using the first primer pair (Table 2). A single-strand linear DNA was prepared from the PCR product with the PyroMark TM Vacuum Prep Workstation (Qiagen). The sequencing PCR was then performed with a gene-specific sequencing primer on a PyroMark Q96 ID System (Qiagen, Hilden, Germany) and analyzed with PyroMark Q CpG software (Qiagen).

### Sequencing of DNMT3A exons 18, 19 and 23:

Mutational analysis was performed for the DNMT3A hotspot region in exon 23, and for the frequently mutated exons 18 and 19 (NM_175629.2). Fifty nanograms of DNA were amplified using PCR primers that had been described before. The PCR product was purified using QIAquick PCR Purification Kit (Qiagen) and Sanger sequencing was performed at MWG (Louisville, KY, USA). qRT-PCR analysis of variant transcripts. Expression of DNMT3A transcripts was analyzed by quantitative real-time PCR (qRT-PCR) using the StepOne Instrument (Applied Biosystems, Darmstadt, Germany). RNA was isolated using the miRNeasy Mini Kit (Qiagen). Quality control and measurement of RNA concentration was carried out with a NanoDrop Spectrophotometer (Thermo Scientific, Wilmington, DE, USA). Total RNA (1 µg) was reverse-transcribed using the high-capacity cDNA Reverse Transcription Kit (Applied Biosystems) and amplified using FAST SYBR Green PCR Master Mix (Applied Biosystems). Because of differential expression of the housekeeping gene (GAPDH) in AML versus healthy control samples (data not presented) and high interindividual variation of DNMT3A expression, we have normalized expression of either transcript 2 or 4 towards the expression of DNMT3A transcripts 1 and 3 (which cannot be distinguished by the primers; Table 3).

### Analysis of DNAm profiles:

For this study, we used publically available data sets of DNAm profiles generated with the HumanMethylation450K BeadChip platform: GSE35069; GSE40699 (Encode project); GSE40279; and TCGA (https://tcga-data.nci.nih.gov/tcga/). β-Values ranging from 0 (non-methylated) to 1 (100% methylation) are provided for each CpG site. The 450K BeadChip comprises 79 CpGs related to the gene DNMT3A. However, in this study we focused on 72 CpGs with β-values in all TCGA samples (missing values might be due to low bead numbers) and these were also considered for graphical presentation. For statistical analysis, we categorized samples as indicated. Raw P-values were then calculated using limma t-test in R/Bioconductor and further adjusted by the Benjamini-Hochberg method. CpG sites with adjusted P<0.05 and an additional cutoff of 20% DNAm change were considered as being differentially methylated. Affiliation of CpG sites with gene regions or CGls was used as described in detail before¹³ and according to UCSC Genome Browser (GRCh37/hg19).

### Analysis of gene expression profiles:

RNA-sequencing data (UNC IlluminaHiSeq_RNASeqV2; n=173) of exon, transcript level and gene level were downloaded from TCGA (https://tcga-data.nci.nih.gov/tcga/). Microarray data (Affymetrix HG-U133_Plus_2; n=183) were also downloaded from TCGA and normalized by RMA in R/Bioconductor. Differentially expressed genes were selected by adjusted P-values using limma t-test (P<0.05) with an additional cutoff for two-fold differential gene expression. Hierarchical clustering (Euclidian distance), principal component analysis and heatmaps were calculated in R. Gene ontology analysis and chemical and genetic perturbations were analysed using the Gene Set Enrichment Analysis tool (http://www.broadinstitute.org/gsea/index.jsp). For analysis of relative expression of different transcripts, we first applied quantile normalization to account for high variation between different transcripts and then normalized their expression to transcript 1.

### Biostatistics on clinical parameters:

Survival data were analyzed using the Kaplan-Meier method and compared using the log-rank test as well as using the multivariate Cox proportional hazard method. Statistical tests were two-sided and were performed using the SAS software package version 9.1.3 (SAS Institute Inc., Cary, NC, USA). For all tests, P<0.05 was used as the level of significance.

### AML samples reveal differentially methylated regions in DNMT3A:

The gene DNMT3A comprises 23 exons and four main transcripts have been described (Figure 4a). A large data set of Hannum and co-workers with 656 blood samples of healthy donors revealed little interindividual variation in the DNAm pattern of CpG sites related to DNMT3A (Figure 4b). In contrast, bone marrow-derived blood samples of 194 AML patients from TCGA repository data revealed three differentially methylated regions (DMRs) within DNMT3A (Figure 4c): in comparison with normal blood, DMR1 was hypomethylated in some AMLs. This hypomethylation revealed relatively little overlap with DNA hypermethylation that occurred at DMR2 and DMR3 (Figure 8). Hypermethylation at DMR2 and DMR3 was also observed in malignant hematopoietic cell lines, whereas different subsets of mature hematopoietic cells displayed relatively little variation, indicating that DNAm changes are not due to changes in cellular composition (Figure 9). DMR2 and DMR3 comprised each a CGI (Figure 4a). Notably, they correspond to an upstream promoter region of transcript 2, which we have recently identified in hematopoietic stem and progenitor cells: particularly DMR2 was non-methylated in CD34+ cells from cord blood and revealed some of the most significant hypermethylation upon culture expansion in vitro (P<10-46). This led us to the assumption that this region is of particular relevance for maintenance of normal hematopoietic integrity and therefore we focused on DNAm at DMR2.

Bisulfite pyrosequencing assays were designed to address specifically DNAm at DMR2 in blood of healthy controls (n=26) and patients with chronic myeloid leukemia (n=15), essential thrombocythemia (n=13), polycythemia vera (n=12), myelofibrosis (n=20) and AML (n=88; Figure 10). As expected, the DNAm level was always below 10% in controls, whereas it was increased in many patients with myeloproliferative neoplasms. Very high DNAm levels at DMR2 were exclusively found in a subset of AML samples (Figure d). Furthermore, it was observed in various cell lines derived from AML (KCL-22, K-562, KYO-1, HEL, MV-4-11, KG-1a and HL-60; Figure 11). DNA hypermethylation was not restricted to individual CpG sites, but seemed to affect the whole CGI and shore region accordingly (Figure 12). Overall, there was no clear association of DNAm at DMR2 with gender and age (Figure 13). We defined epimutations to be present if the DNAm level at the CpG site related to probe set cg08485187 was above 10% as this level has not been reached in any of the controls. Notably, 15% of AML samples even revealed a DNAm level of more than 50%, indicating that the epimutation affected both alleles-particularly given that not all cells derive from the malignant clone. When we analyzed DNAm at cg08485187 in relation to the percentage of blast cells, it was evident that there was no linear relationship between blast counts and DNAm at DMR2 (Figure 14). This supported the notion that AML samples can be discerned in those with or without epimutation in DNMT3A.

### Hypermethylation in DMR2 is higher in samples without genetic mutation in DNMT3A:

In several other tumor-associated genes, such as BRCA1 in ovarian cancer and CDKN2A in squamous carcinoma, the causal relevance of epigenetic changes has been supported by the finding that such silencing events are mutually exclusive with mutational inactivation of the same gene. To analyze if DNA hypermethylation at DMR2 is related to genomic mutations of DNMT3A, we sequenced the exons 18, 19 and 23 in our AML samples. Three patients had mutations at the hotspot Arg882, one patient revealed a novel mutation at Asn879 and one patient had a stop mutation at Cys710. With our assays, we found genetic mutations exclusively in patients without epimutations, but this was not significant because of the relatively low sample number and because of the restriction of mutational analysis to exons 18, 19 and 23 (Figure 15). However, the trend was also observed in the 194 AML samples of TCGA, which provided sequencing information of the entire DNMT3A gene:9 epimutations and mutations in DNMT3A appeared to be vastly mutually exclusive (P=0.015, two-sided Fisher's exact test; Figure 5a). Furthermore, the DNAm levels within DMR2 of DNMT3A were significantly higher in samples without genetic mutation (P=0.0037, Wilcoxon's rank-sum test; Figure 5b). Patients harboring mutations were enriched in the cohorts with intermediate or poor cytogenetic risk as described before (P<10-5; two-sided Fisher's exact test). Interestingly, patients with epimutations in DNMT3A-even in the subset without mutations-revealed the same enrichment in intermediate- or poor cytogenetic-risk groups (P<0.0002; Figure 5c) and the same tendency was also observed in our patients (Figure 15d). In contrast, aberrant hypomethylation at DMR1 was associated with favorable cytogenetic risk (Figure 16). In analogy to DNMT3A mutations, the epimutations were not found in core-binding factor-AMLs (defined by RUNX1-RUNX1T1 or CBFB-MYH11 fusion) and only very rarely in acute promyelocytic leukemia (PML-PARA fusion). On the other hand, mutations as well as epimutations in DNMT3A were highly significantly associated with mutations of IDH1, IDH2, RUNX1 and NPM1 (Figure 17).

### Epimutations and mutations in DNMT3A downregulate relative expression of DNMT3A transcript 2:

Hypermethylation at DMR2 and DMR3, which seem to be related to the internal promoter region of transcript 2, may result in the downregulation of DNMT3A transcript 2. In fact, analysis of RNA-sequencing data of TCGA9 revealed that particularly those exons included in transcript 2 were highly expressed in AML samples (Figure 5d). Interestingly, they were slightly less expressed in patients with epimutation (Figure 18a). Because of the high interindividual variation in DNMT3A expression, we have determined relative expression as compared with transcript 1. In fact, transcript 2 was then slightly less expressed in patients with epimutation in DNMT3A (P=0.026) and the same tendency was also observed in our AML samples using quantitative real-time PCR (qRT-PCR) (Figures 18c-e). Unexpectedly, an even more pronounced downregulation of transcript 2 was observed in patients with mutations in DNMT3A, even though the frequently mutated exon 23 is usually also part of transcripts 1 and 3 (Figure 5d). In this regard, it is also interesting that all genomic mutations in DNMT3A described in TCGA data were associated with exons of transcript 2, but not with DMR2 or DMR3 directly (Figure 4c).

### DNAm changes associated with DNMT3A epimutations:

Next, we analyzed if the DNMT3A epimutation has a positive or negative correlation with DNAm changes: 3536 CpGs revealed significant DNAm changes (samples with DNMT3A mutation were excluded for this comparison; Figure 19). Notably, almost the entire set (3523 CpGs) was hypermethylated in samples with DNMT3A epimutation. In contrast, comparison of DNAm profiles of AMLs with DNMT3A mutation versus wild-type AMLs (both without epimutation) revealed that only 78 CpGs were significantly hypermethylated, whereas 2595 CpGs were hypomethylated in mutated samples (Figure 20) and many of them were associated with homeobox genes, including HOXA2, A6, A7, A9, B2, B3, B4 and B8. The association of differential DNAm in samples with either mutation or epimutation was moderate, but overall there seemed to be a significant relationship (P=0.0005, _{X}2; Figure 21). When epimutated and mutated samples were grouped together for comparison with samples without DNMT3A modifications, we detected 444 CpGs as differentially methylated (Figures 6a and b and Table 4). These results revealed some concordance in DNAm changes upon either epimutation or mutation of DNMT3A and this was particularly observed in hypomethylated CpGs. Such overlapping hypomethylation was associated with several homeobox genes including in HOXA9 (Figure 6c).

### Gene expression changes associated with DNMT3A epimutations:

We have then analyzed if DNAm changes in AML with DNMT3A epimutation are also reflected on gene expression level. RNA-sequencing data from the TCGA9 revealed that 205 genes were differentially expressed in AML samples with or without DNMT3A epimutation (samples with DNMT3A mutation were excluded from this comparison): 81 genes were significantly downregulated, whereas 124 genes were upregulated, the latter including HOXA1, A2, A3, A4, A5, A6, A7, A9, A10, B3, B4, B5 and B6. Chemical and genetic perturbations (CGP) demonstrated most significant association of upregulated genes with three data sets of AML mutations in NPM1 (P-values always <10-16), which might point to an association of DNMT3A epimutation with NPM1 mutation as mentioned above. Furthermore, differentially expressed genes were also found to be upregulated in hematopoietic stem and progenitor cells upon overexpression of NUP98-HOXA9 fusion protein (P<10-16). Heatmap analysis and principal component analysis supported the notion that differential gene expression in AML samples with epimutation is related to differential gene expression in samples with mutations in DNMT3A (Figure 22). For example, HOXA9 is significantly upregulated in both categories of DNMT3A modifications and this has been associated with poor prognosis in AML before (Figure 6d). In fact, almost the entire HOXA and HOXB cluster were significantly upregulated in AML samples with either epimutation or mutation in DNMT3A. The clear correlation of differentially expressed genes indicates that the molecular sequel of epimutation and mutation in DNMT3A might be related (Figure 6e). On the other hand, a significant compensatory upregulation in gene expression of DNMT1, DNMT3B or DNMT3L was not observed (Figure 23).

### DNMT3A epimutations are associated with poor prognosis:

Finally, we analyzed the impact of DNMT3A epimutations on clinical prognosis. Indeed, hypermethylation within the gene DNMT3A was associated with significantly shorter event-free survival (Figure 7) and shorter OS (Figure 24) in the data set of TCGA. However, such a pronounced effect could not be recapitulated in our own data set, which can likely be attributed to the relatively small set of 88 AML samples. Furthermore, it is not clear if the prognostic relevance is independent of cytogenetic risk analysis: when we only considered samples from TCGA with intermediate- or poor-risk score, the results were no more significant (Figure 24e). Thus, larger data sets might be required to determine whether or not epimutations in DNMT3A have independent diagnostic relevance. Either way, the results support the notion that epimutations in DNMT3A, just as genomic mutations, are rather associated with poor-risk score and poor prognosis in AML.

Epimutations define heritable changes in gene activity, which, unlike classical gene mutations, are not due to changes in DNA sequence. They occur in somatic cells, particularly in tumor suppressor genes. RB methylation was identified as the first example of epimutation and subsequently many other oncogenes have been shown to be methylated in sporadic cancers. In this study, we describe that AML patients often reveal aberrant hypermethylation in DNMT3A and the same effect was also observed in AML cell lines. We used an absolute threshold of 10% DNAm level to select patients with epimutations-this threshold was not reached in controls and other authors used even lower cutoffs to define epimutations. On the other hand, microarray data as well as bisulfite pyrosequencing indicated that the methylation level was sometimes even above 50%. With regard to the heterogeneous composition of malignant and normal cells, this indicates that the epimutation is homozygous in these cases. This might either be due to uniparental disomy, selection pressure or to a related upstream mechanism that entails the epimutation on both alleles of DNMT3A. Notably, very high aberrant DNA hypermethylation was only observed in AML samples, but not in chronic myeloid leukemia or other myeloproliferative neoplasms. It might be a common phenomenon that epimutations are disease specific if the corresponding genomic mutations are specifically enriched in this disease, too.

We have recently analyzed DNAm changes upon culture expansion of hematopoietic stem and progenitor cells in vitro: the same genomic region of DNMT3A as described in this study was hardly methylated in freshly isolated CD34+ cells from cord blood but it was significantly hypermethylated in culture-expanded CD34+cells (P<10⁻⁴⁶) and this was associated with downregulation of transcripts 2 and 4. Transcript 4 does not possess methyltransferase activity, but downregulation may still be functionally relevant if it contributes to regulatory complexes. This led us to the assumption that variant transcripts of DNMT3A are important for regulation of self-renewal versus differentiation. Spliceosome assembly occurs at the same time as transcription and therefore the DNA structure can directly influence alternative splicing. It has been shown that a DNA-binding protein, CCCTC-binding factor (CTCF), can promote inclusion of weak upstream exons by mediating local RNA polymerase II pausing. Notably, a CTCF binding site is also located within DMR2 of DNMT3A and hence the epimutation may be relevant for regulation of alternative splicing. In fact, the epimutation is associated with moderate downregulation of transcripts 2 and 4 when considering the relative expression towards transcript 1. These results indicate that the epimutation has impact on expression of variant transcripts but it does not necessarily indicate functional competition of corresponding proteins. Unexpectedly, a much more pronounced downregulation of transcript 2 was observed in samples with genomic mutations in DNMT3A. It is conceivable that mutations as well as DNAm changes influence alternative splicing and/or the stability of DNMT3A transcript 2. Either way, it is striking that epimutations and mutations of DNMT3A seem to have similar consequences on the relative expression of this transcript. So far, there is little knowledge about the specific functions of variant transcripts, their stability and their impact on the DNAm profile-yet, it is well possible that the various isoforms of DNMT3A have different sequence specificity or interact with different partners.

Loss of DNAm activity of mutated DNMT3A has been demonstrated before whereas hypermethylation upon epimutation of DNMT3A appears to be paradoxical. This might be due to the fact that the mutations within the methyltransferase domain have impact on all active transcripts, whereas the epimutations rather influence relative expression-and potentially alternative splicing-of specific transcripts. On the other hand, hypermethylation has also been described in inducible conditional Dnmt3a-knockout mice, indicating that dysregulation of this methyltransferase causes widespread indirect changes. The opposite effects of epimutations and mutations on DNAm changes did not refer to the same CpG sites. Overall, there was even a moderate association in DNAm changes. Furthermore, there was a clear correlation of differentially expressed genes. Other groups have previously demonstrated multiple expression clusters associated with DNMT3A mutations but none of them were clearly defined by the mutation status. Our results now suggest that this might be attributed to epimutations in DNMT3A conferring similar changes in gene expression pattern. Extensive upregulation of HOXA7 and HOXB2, B3, B4, B5, B6, B7 and B8 has been demonstrated before in AML samples with mutation in DNMT3A. In analogy, many genes of the HOXA and HOXB cluster were upregulated in samples with an epimutation in DNMT3A. HOX genes have a crucial role in regulation of normal hematopoiesis and they appear to be involved in pathogenesis of AML and other cancers. It is therefore conceivable that the molecular sequel of DNMT3A mutations/epimutations on regulation of HOX genes have a central role for disease progression.

The DNAm level at DMR2 was significantly higher in AML samples without epimutation, but both modifications were significantly associated with mutations of IDH1, IDH2, RUNX1 and NPM1. Epimutations and mutations in DNMT3Awere enriched in AML samples with an intermediate or poor cytogenetic risk. Wakita and co-workers have recently suggested that an initial mutation in an epigenetic-modifying gene-such as DNMT3A-may entail genetic instability leading to resistance to therapy and relapse. A similar effect might also apply for epimutations. Furthermore, analysis of TCGA data revealed that both of them are associated with significantly shorter event-free survival. These findings further substantiate the perception that DNMT3A epimutations mimic genetic mutations of DNMT3A, and they suggest that both mutations synergize with IDH1/2 mutations, RUNX1 mutations and NPM1 mutations to alter DNAm, histone modification, hematopoietic differentiation and cell survival. It is conceivable that DNMT3A modifications represent early events-potentially even initiating events-that then entail the less frequent mutations in IDH1, IDH2, RUNX1 and NPM1. However, this hierarchy of clonal development needs to be further analyzed. On the other hand, epimutations and mutations in DNMT3A were not observed in core-binding factor-AMLs and hardly in acute promyelocytic leukemias and this supports the notion that these entities have different pathophysiologic origin.

Analysis of the epimutation in DNMT3A is based on DNAm at a single CpG site-yet, differential DNAm at neighboring CpGs within the CPI revealed a very high correlation. Considering that bisulfite pyrosequencing is a relatively inexpensive high-throughput technique and that our assay is site specific, screening of larger patient populations is feasible. This approach might be useful for risk stratification and it might also be relevant for therapeutic decisions: cytosine nucleoside analogs-such as azacitidine and decitabine-are approved for the treatment of AML. They are incorporated into the DNA and bind covalently to catalytic sites of all DNMTs. It is conceivable that DNMT inhibitors are particularly effective in patients with epimutations in DNMT3A as they reflect aberrant DNA hypermethylation at many CpG sites. Furthermore, it has been demonstrated that in patients with genomic mutations in DNMT3A the relative resistance to chemotherapy can be overcome with high-dose daunorubicin treatment, which then improved survival rates. It needs to be assessed if the epimutation in DNMT3A has a significant effect on the outcome with dose-intensive chemotherapy, too.

The observation that about half of the AML patients either have a mutation or an epimutation in DNMT3A indicates a high relevance for disease development. Despite the very different nature of these epigenetic or genetic aberrations, their molecular and functional sequels appear to be related. The bisulfite pyrosequencing assay described here provides a relatively simple and cost-effective approach to screen for aberrant DNA hypermethylation within DNMT3A. The impact for diagnosis, risk stratification, disease monitoring and choice of therapeutic regimens needs to be analyzed further in independent data sets-yet both mutations and epimutations in DNMT3A should both be taken into account.

**Table 1: Characteristics of the patient cohort.**

| **Parameter** | **amount** |
|---|---|
| number of patient | 88 |
| age, years (median and range) | 58 (18 - 89) |

| gender | |
|---|---|
| male | 40 |
| female | 48 |

| FAB subtype | |
|---|---|
| M0 | 12 |
| M1 | 24 |
| M2 | 12 |
| M3 | 3 |
| M4 | 29 |
| M4Eo | 3 |
| M5 | 5 |

| karyotype | |
|---|---|
| favorable (CBF leukemia) | 14 |
| intermediate | 43 |
| adverse | 23 |
| no data | 8 |

| laboratory parameters (median and range) | |
|---|---|
| WBC (G/l) | 30.5 (0.8 - 294.6) |
| hemoglobin (g/l) | 96.2 (68 - 163) |
| platelet count (G/l) | 118 (7 - 782) |
| LDH (UI/l) | 642 (124 - 7662) |
| preexisting myelodysplastic syndrome | 19 |
| induction chemotherapy | 61 |

| | |
|---|---|
| FAB, French-American-British; CBF, core binding factor; WBC, white blood cell; LDH, lactate dehydrogenase | |

**Table 2: Primers for Pyrosequencing.**

| **Primer** | **Sequence** |
|---|---|
| assay 1 forward | 5'-GGTTTGGGTTTATTGTAGGAAGGTTATTAAGGT-3' (SEQ ID NO: 3) |
| assay 1 reverse | 5'-AATCCAAAACCCCCCTATCACGAAA-Bio-3' (SEQ ID NO: 4) |
| assay 1 sequencing | 5'-GTGGGGGGAGATAAA-3' (SEQ ID NO: 5) |
| assay 2 forward | 5'-Bio-TTAGGTTCGGGAGTATTAGGGGGAGG-3' (SEQ ID NO: 6) |
| assay 2 reverse | 5'-AACAAAACCAAACAAAATAAATAAAACAACCC-3' (SEQ ID NO: 7) |
| assay 2 sequencing | 5'-AAATAAAACAACCCTTAAAA-3' (SEQ ID NO: 8) |

**Table 3: Primers for qRT-PCR.**

| **Primer** | **Sequence** |
|---|---|
| DNMT3A tr. 1&3 forward | 5'-ACCCTGCCTGAAGCCTCAAG-3' (SEQ ID NO: 11) |
| DNMT3A tr. 1&3 reverse | 5'-AAGGTGAGCCTCGGCATGG-3' (SEQ ID NO: 12) |
| DNMT3A tr. 2 forward | 5'-GTGGATCGTAGCCTGAAAG-3' (SEQ ID NO: 13) |
| DNMT3A tr. 2 reverse | 5'-TGGTGGCATTCTTGTCC-3' (SEQ ID NO: 14) |
| DNMT3A tr. 4 forward | 5'-AAGCGGGTGAGTCCTCAGC-3' (SEQ ID NO: 15) |
| DNMT3A tr. 4 reverse | 5'-CATATGCGCAGGCTGCATCC-3' (SEQ ID NO: 16) |
| GAPDH forward | 5'-TTCGTCATGGGTGTGAAC-3' (SEQ ID NO: 17) |
| GAPDH reverse | 5'-CTGTGGTCATGAGTCCTT-3' (SEQ ID NO: 18) |

### Non-patent literature

1. Challen GA, Sun D, Jeong M et al. Dnmt3a is essential for hematopoietic stem cell differentiation. Nat.Genet. 2012;44:23-31.
2. Ley TJ, Ding L, Walter MJ et al. DNMT3A mutations in acute myeloid leukemia. N.Engl.J.Med. 2010;363:2424-2433.
3. Ley TJ, Miller C, Ding L et al. Genomic and epigenomic landscapes of adult de novo acute myeloid leukemia. N Engl J Med 2013
4. Walter MJ, Ding L, Shen D et al. Recurrent DNMT3A mutations in patients with myelodysplastic syndromes. Leukemia 2011;25:1153-1158.
5. Yamashita Y, Yuan J, Suetake I et al. Array-based genomic resequencing of human leukemia. Oncogene 2010;29:3723-3731.
6. Yan XJ, Xu J, Gu ZH et al. Exome sequencing identifies somatic mutations of DNA methyltransferase gene DNMT3A in acute monocytic leukemia. Nat.Genet. 2011;43:309-315.
7. Ribeiro AF, Pratcorona M, Erpelinck-Verschueren C et al. Mutant DNMT3A: a marker of poor prognosis in acute myeloid leukemia. Blood 2012;119:5824-5831.
8. Hayette S, Thomas X, Jallades L et al. High DNA methyltransferase DNMT3B levels: a poor prognostic marker in acute myeloid leukemia. PLoS ONE 2012;7:e51527.
9. Hannum G, Guinney J, Zhao L et al. Genome-wide Methylation Profiles Reveal Quantitative Views of Human Aging Rates. Mol.Cell 2013;49:459-367.
10. Reinius LE, Acevedo N, Joerink M et al. Differential DNA methylation in purified human blood cells: implications for cell lineage and studies on disease susceptibility. PLoS ONE 2012;7:e41361.
11. Jourdan E, Boissel N, Chevret S et al. Prospective evaluation of gene mutations and minimal residual disease in patients with core binding factor acute myeloid leukemia. Blood 2013;121:2213-2223.
12. Marcucci G, Metzeler KH, Schwind S et al. Age-related prognostic impact of different types of DNMT3A mutations in adults with primary cytogenetically normal acute myeloid leukemia. J Clin.Oncol. 2012;30:742-750.
13. Bibikova M, Barnes B, Tsan C et al. High density DNA methylation array with single CpG site resolution. Genomics 2011;98:288-295.
14. Stegelmann F, Bullinger L, Schlenk RF et al. DNMT3A mutations in myeloproliferative neoplasms. Leukemia 2011;25:1217-1219.

## Claims

1. Method for determining a human individual's predisposition to contract a hematopoietic malignant myeloid disease, wherein the myeloid disease is acute myeloid leukemia (AML) or myelodysplastic syndrome (MDS), comprising:
- Providing at least one nucleic acid of the human individual;
- Determining a methylation level of at least one specific region of the nucleic acid comprising at least one CpG-dinucleotide, wherein the specific region consists of the gene of the DNA-methyltransferase DNMT3A; and
- Comparing the determined methylation level with at least one reference methylation level of the specific region(s), wherein the risk of the human individual to contract the myeloid disease is increased if the determined methylation level is increased compared to the reference methylation level.

2. Method for evaluating a human individual's response to a therapy for treating a hematopoietic malignant myeloid disease, wherein the myeloid disease is acute myeloid leukemia (AML) or myelodysplastic syndrome (MDS), and wherein the therapy has been performed by administering at least one DNA-methyltransferase inhibitor or at least one anthracycline antibiotic, comprising:
- Providing at least one first nucleic acid of the human individual;
- Determining a first methylation level of at least one specific region of the first nucleic acid comprising at least one specific CpG-dinucleotide, wherein the specific region consists of the gene of the DNA-methyltransferase DNMT3A;
- Providing at least one second nucleic acid of the human individual after the therapy was performed;
- Determining a second methylation level of the specific region(s) of the second nucleic acid, wherein the second methylation level is a methylation level of the same specific CpG-dinucleotide; and
- Comparing the first methylation level with the second methylation level, wherein the therapy has a positive effect on the myeloid disease if the second methylation level is decreased compared to the first methylation level.

3. Method according to claim 2, wherein the DNA-methyltransferase inhibitor is azacitidine or decitabine, or wherein the anthracycline antibiotic is daunorubicine or idarubicine.

4. Method according to any one of claims 1 to 3, wherein the specific region comprises at least one promoter region.

5. Method according to any one of claims 1 to 4, wherein the specific region comprises the nucleotide sequence of SEQ ID NO: 1.

6. Method according to any one of claims 1, 4 and 5, wherein the determined methylation level is 10 % or higher.

7. Method according to any one of claims 1 to 6, wherein the DNA is derived from blood cells selected from the group consisting of monocytes, T cells, hematopoietic progenitor cells (HPCs), lymphocytes, mononuclear cells (MNCs), and leucocytes.

## Patentansprüche

1. Verfahren zum Ermitteln der Veranlagung eines menschlichen Individuums an einer hämatopoietischen malignen Myelomerkrankung zu erkranken, wobei die Myelomerkrankung eine akute myeloische Leukämie (AML) oder ein myelodysplastisches Syndrom (MDS) ist, umfassend:
- Bereitstellen mindestens einer Nukleinsäure des menschlichen Individuums;
- Bestimmen eines Methylierungsgrades mindestens einer spezifischen Region der Nukleinsäure, welche mindestens ein CpG-Dinukleotid umfasst, wobei die spezifische Region aus dem Gen der DNA-Methyltransferase DNMT3A besteht; und
- Vergleichen des bestimmten Methylierungsgrades mit mindestens einem Referenz-Methylierungsgrad der spezifischen Region(en), wobei das Risiko des menschlichen Individuums, an der Myelomerkrankung zu erkranken, erhöht ist, wenn der bestimmte Methylierungsgrad im Vergleich zum Referenz-Methylierungsgrad erhöht ist.

2. Verfahren zum Abschätzen des Ansprechens eines menschlichen Individuums auf eine Therapie zur Behandlung einer hämatopoietischen malignen Myelomerkrankung, wobei die Myelomerkrankung eine akute myeloische Leukämie (AML) oder ein myelodysplastisches Syndrom (MDS) ist, und wobei die Therapie durch die Verabreichung mindestens eines DNA-Methyltransferase-Inhibitors or mindestens eines Anthracyclin-Antibiotikums durchgeführt wurde, umfassend:
- Bereitstellen mindestens einer ersten Nukleinsäure des menschlichen Individuums;
- Bestimmen eines ersten Methylierungsgrades mindestens einer spezifischen Region der ersten Nukleinsäure, welche mindestens ein spezifisches CpG-Dinukleotid umfasst, wobei die spezifische Region aus dem Gen der DNA-Methyltransferase DNMT3A besteht;
- Bereitstellen mindestens einer zweiten Nukleinsäure des menschlichen Individuums nachdem die Therapie durchgeführt wurde;
- Bestimmen eines zweiten Methylierungsgrades der spezifischen Region(en) der zweiten Nukleinsäure, wobei der zweite Methylierungsgrad ein Methylierungsgrad des gleichen spezifischen CpG-Dinukleotids ist; und
- Vergleichen des ersten Methylierungsgrads mit dem zweiten Methylierungsgrad, wobei die Therapie eine positive Wirkung auf die Myelomerkrankung hat, wenn der zweite Methylierungsgrad im Vergleich zum ersten Methylierungsgrad verringert ist.

3. Verfahren nach Anspruch 2, wobei der DNA-Methyltransferase-Inhibitor Azacitidin or Decitabin ist, oder wobei das Anthracyclin-Antibiotikum Daunorubicin or Idarubicin ist.

4. Verfahren nach einem der Ansprüche 1 bis 3, wobei die spezifische Region mindestens eine Promotor-Region umfasst.

5. Verfahren nach einem der Ansprüche 1 bis 4, wobei die spezifische Region die Nukleotid-Sequenz gemäß SEQ ID NO: 1 umfasst.

6. Verfahren nach einem der Ansprüche 1, 4 und 5, wobei der bestimmte Methylierungsgrad 10 % oder höher ist.

7. Verfahren nach einem der Ansprüche 1 bis 6, wobei die DNA aus Blutzellen stammt, die ausgewählt sind aus der Gruppe bestehend aus Monozyten, T-Zellen, hämatopoietischen Vorläuferzellen (HPCs), Lymphozyten, mononuklearen Zellen (MNCs) und Leukozyten.

## Revendications

1. Procédé de détermination de la prédisposition d'un individu humain à contracter une maladie myéloïde hématopoïétique maligne, dans lequel la maladie myéloïde est la leucémie myéloïde aiguë (LMA) ou le syndrome myélodysplasique (MDS), comprenant :
- la fourniture d'au moins un acide nucléique de l'individu humain ;
- la détermination d'un niveau de méthylation d'au moins une région spécifique de l'acide nucléique comprenant au moins un dinucléotide CpG, dans lequel la région spécifique est constituée du gène de l'ADN méthyltransférase DNMT3A ; et
- la comparaison du niveau de méthylation déterminé à au moins un niveau de méthylation de référence de la (des) région(s) spécifique(s), dans lequel le risque que l'individu humain contracte la maladie myéloïde est accru si le niveau de méthylation déterminé est plus élevé comparé au niveau de méthylation de référence.

2. Procédé d'évaluation de la réponse d'un individu humain à une thérapie pour le traitement d'une maladie myéloïde hématopoïétique maligne, dans lequel la maladie myéloïde est la leucémie myéloïde aiguë (LMA) ou le syndrome myélodysplasique (MDS), et dans lequel la thérapie a été réalisée par administration d'au moins un inhibiteur de l'ADN-méthyltransférase ou d'au moins un antibiotique anthracycline, comprenant :
- la fourniture d'au moins un premier acide nucléique de l'individu humain ;
- la détermination d'un premier niveau de méthylation d'au moins une région une région spécifique du premier acide nucléique comprenant au moins un dinucléotide CpG, dans lequel la région spécifique est constituée du gène de l'ADN méthyltransférase DNMT3A ;
- la fourniture d'au moins un second acide nucléique de l'individu humain après la réalisation de la thérapie ;
- la détermination d'un second niveau de méthylation de la (des) région(s) spécifique(s) du second acide nucléique, dans lequel le second niveau de méthylation est un niveau de méthylation du même dinucléotide CpG spécifique ; et
- la comparaison du premier niveau de méthylation au second niveau de méthylation, dans lequel la thérapie a un effet positif sur la maladie myéloïde si le second niveau de méthylation est réduit comparé au premier niveau de méthylation.

3. Procédé selon la revendication 2, dans lequel l'inhibiteur de l'ADN méthyltransférase est l'azacitidine ou la décitabine, ou dans lequel l'antibiotique anthracycline est la daunorubicine ou l'idarubidine.

4. Procédé selon l'une quelconque des revendications 1 à 3, dans lequel la région spécifique comprend au moins une région de promoteur.

5. Procédé selon l'une quelconque des revendications 1 à 4, dans lequel la région spécifique comprend la séquence nucléotidique SEQ ID NO : 1.

6. Procédé selon l'une quelconque des revendications 1, 4 et 5, dans lequel le niveau de méthylation déterminé est de 10 % ou plus.

7. Procédé selon l'une quelconque des revendications 1 à 6, dans lequel l'ADN est dérivé de cellules sanguines sélectionnées dans le groupe constitué par les monocytes, les lymphocytes T, les cellules progénitrices hématopoïétiques (CPH), les lymphocytes, les cellules mononucléaires (CMN), et les leucocytes.


### SEQUENCE LISTING

<110> Rheinisch-Westfälische Technische Hochschule (RWTH) Aachen
<120> Method for determining a human individual's predisposition to contract a malignant disease
<130> 13419WO
<150> EP 13167411.1
   <151> 2013-05-13
<160> 18
<170> PatentIn version 3.5
<210> 1
   <211> 650
   <212> DNA
   <213> Homo sapiens
<400> 1
   ttgctggagg tggggaagtg agccctcctt ccacctgggc ccctgagctt ctttgtcggg        60
   aaccgacagc gtatcgggcc tgggcccact gcaggaaggt cactaaggct ggctcatggt       120
   agcttggggt ggggggagac aaagagcctg aaacggaaaa aacagccccg acttcatctc        180
   ctggctgtga agcggaagcc agccacagtg ttcacgccgc gggtcctcag cccgggaggc       240
   cactcccttc cctcgtgaca ggggggtcct ggactggttt tgggctgacc ccgggcgccc        300
   aggacagcgc tgtcctccgt ggttctgtcc ctggtgacag cgactcccgc tagctaggcc       360
   cgggagcacc agggggaggg aggcaggagg gccaccgtgg gtgcgggcct ggcgccgggc       420
   tcagcccgcg ggggccgcgt gggcggggac ggggcgtctt gtcgcgggca cctgtgcgcg       480
   tgggcgggcg cgcactcgcc agcgctttgt tcgtgaccgg ccttttaagg gctgtctcac       540
   ccatcttgtc tggctctgcc tccctgtttc ctttccgtcc tctctcccac cacagccagc       600
   tccccacttt tttcgcgggg gccccctcca gcctgtccgg ggcctccccg                  650
<210> 2
   <211> 122
   <212> DNA
   <213> Homo sapiens
<400> 2
   ctctcaccac agattgcacc tctgcctggg ggcgccactg ggctcatcag actgagctca        60
   cggtgccccc tggtgggcga tgccagtggc gccagtgcag cttggagccc gcaatacaag       120
   gt                                                                      122
<210> 3
   <211> 33
   <212> DNA
   <213> Artificial Sequence
<220>
   <223> Assay 1, PCR forward primer for pyrosequencing
<400> 3
   ggtttgggtt tattgtagga aggttattaa ggt 33
<210> 4
   <211> 25
   <212> DNA
   <213> Artificial Sequence
<220>
   <223> Assay 1, PCR reverse primer for pyrosequencing
<400> 4
   aatccaaaac ccccctatca cgaaa 25
<210> 5
   <211> 15
   <212> DNA
   <213> Artificial Sequence
<220>
   <223> Assay 1, sequencing primer
<400> 5
   gtggggggag ataaa 15
<210> 6
   <211> 26
   <212> DNA
   <213> Artificial Sequence
<220>
   <223> Assay 2, PCR forward primer for pyrosequencing
<400> 6
   ttaggttcgg gagtattagg gggagg 26
<210> 7
   <211> 32
   <212> DNA
   <213> Artificial Sequence
<220>
   <223> Assay 2, PCR reverse primer for pyrosequencing
<400> 7
   aacaaaacca aacaaaataa ataaaacaac cc 32
<210> 8
   <211> 20
   <212> DNA
   <213> Artificial Sequence
<220>
   <223> Assay 2, sequencing primer
<400> 8
   aaataaaaca acccttaaaa 20
<210> 9
   <211> 125
   <212> DNA
   <213> Homo sapiens
<400> 9
   gagtttgaaa cggaaaaaat agtttcgatt ttattttttg gttgtgaagc ggaagttagt 60
   tatagtgttt acgtcgcggg tttttagttc gggaggttat tttttttttt cgtgataggg 120
   gggtt 125
<210> 10
   <211> 157
   <212> DNA
   <213> Homo sapiens
<400> 10
   aaccgatcac gaacaaaacg ctaacgaata cgcgcccgcc cacgcgcaca aatacccgcg 60
   acaaaacgcc ccatccccgc ccacgcgacc cccgcgaact aaacccgacg ccaaacccgc 120
   acccacgata accctcctac ctccctcccc ctaatac 157
<210> 11
   <211> 20
   <212> DNA
   <213> Artificial Sequence
<220>
   <223> Forward primer for qRT-PCR
<400> 11
   accctgcctg aagcctcaag 20
<210> 12
   <211> 19
   <212> DNA
   <213> Artificial Sequence
<220>
   <223> Reverse primer for qRT-PCR
<400> 12
   aaggtgagcc tcggcatgg 19
<210> 13
   <211> 19
   <212> DNA
   <213> Artificial Sequence
<220>
   <223> Forward primer for qRT-PCR
<400> 13
   gtggatcgta gcctgaaag 19
<210> 14
   <211> 17
   <212> DNA
   <213> Artificial Sequence
<220>
   <223> Reverse primer for qRT-PCR
<400> 14
   tggtggcatt cttgtcc 17
<210> 15
   <211> 19
   <212> DNA
   <213> Artificial Sequence
<220>
   <223> Forward primer for qRT-PCR
<400> 15
   aagcgggtga gtcctcagc 19
<210> 16
   <211> 20
   <212> DNA
   <213> Artificial Sequence
<220>
   <223> Reverse primer for qRT-PCR
<400> 16
   catatgcgca ggctgcatcc 20
<210> 17
   <211> 18
   <212> DNA
   <213> Artificial Sequence
<220>
   <223> Forward primer for qRT-PCR
<400> 17
   ttcgtcatgg gtgtgaac 18
<210> 18
   <211> 18
   <212> DNA
   <213> Artificial Sequence
<220>
   <223> Reverse primer for qRT-PCR
<400> 18
   ctgtggtcat gagtcctt 18